# EUROPEAN PATENT SPECIFICATION

(11) **EP 2 639 216 B1**
(45) Date of publication and mention of the grant of the patent: **11.07.2018**
(21) Application number: 11839101.0
(22) Date of filing: 08.11.2011
(51) Int. Cl.: C07C 29/17, C07C 45/63, C07C 45/65, C12P 7/22, C12P 7/38, C07B 53/00, C07C 45/62, C07D 241/04

(54) **HALOGENATED INDENONES AND METHOD FOR PRODUCING OPTICALLY ACTIVE INDANONES OR OPTICALLY ACTIVE INDANOLS BY USING SAME**
HALOGENIERTE INDENONE UND VERFAHREN ZUR HERSTELLUNG OPTISCH AKTIVER INDANONE ODER OPTISCH AKTIVER INDANOLE UNTER VERWENDUNG DIESER
INDÉNONES HALOGÉNÉES ET PROCÉDÉ POUR LA PRODUCTION D'INDANONES OPTIQUEMENT ACTIFS OU D'INDANOLES OPTIQUEMENT ACTIFS UTILISANT LESDITES INDÉNONES

(30) Priority: 09.11.2010 JP 2010251095
(43) Date of publication of application: 18.09.2013
(73) Proprietor: Kaneka Corporation, Osaka-shi, Osaka 530-8288 (JP)
(72) Inventor: YAMAMOTO, Shohei, Takasago-shi Hyogo 676-8688 (JP); NISHIHACHIJO, Masakatsu, Takasago-shi Hyogo 676-8688 (JP); TANAKA, Rie, Takasago-shi Hyogo 676-8688 (JP); KAWANO, Shigeru, Takasago-shi Hyogo 676-8688 (JP); FUJII, Akio, Takasago-shi Hyogo 676-8688 (JP)
(74) Representative: Vossius & Partner Patentanwälte Rechtsanwälte mbB
(86) International application number: PCT/JP2011/075767
(87) International publication number: WO 2012/063843

(56) References cited:
- WO-A1-2006/129628
- WO-A1-2009/036275
- JP-A- 2003 519 207
- JP-A- 2007 502 784
- JP-A- 2008 530 038
- S.C. PAKRASHI ET AL.: "The Reformatsky reaction on 1-naphthyl-2-bromophenyl ketone", TETRAHEDRON, vol. 18, 1962, pages 1243-1249, XP002721656,
- JAMES F. FREEMAN ET AL.: "3-Phenylindones. I. The synthesis of 6-chloro-3-(p-chlorophenyl)-1-indenone and some related compounds", J. AM. CHEM. SOC., vol. 72, 1950, pages 1522-1526, XP002721657,
- WILLIAM M. C. ET AL.: 'A Highly Enantioselective Conjugate Reduction of 3-Arylinden-l-ones Using Bakers' Yeast for the Preparation of (S)-3- Arylindan-1-ones' ORGANIC LETTERS vol. 1, no. 11, 1999, pages 1839 - 1842, XP002951487
- CHRISTIAN H. ET AL.: 'Catalytic Asymmetric Total Synthesis of the Muscarinic Receptor Antagonist (R)-Tolterodine' ADV. SYNTH. CATAL. vol. 347, 2005, pages 662 - 666, XP008131820
- JAMES F. F. ET AL.: '3-Phenylindones. I. The Synthesis of 6-Chloro-3-(p-chlorophenyl)-l- indenone and Some Related Compounds' J. AM. CHEM. SOC. vol. 72, 1950, pages 1522 - 1526, XP002721657
- S. C. PAKRASHI ET AL.: 'The Reformatsky Reaction on 1-Naphthyl-2-Bromophenyl Ketone' TETRAHEDRON vol. 18, 1962, pages 1243 - 1249, XP002721656

## Description

### TECHNICAL FIELD

The present invention relates to a technology for producing optically active indanones and optically active indanols (preferably optically active aryl indanones and optically active aryl indanols, and their derivatives).

### BACKGROUND ART

Among optically active aryl indanol derivatives, for example, optically active 6-chloro-3-phenylindanol is an useful compound for producing trans-4-((1R,3S)-6-chloro-3-phenylindan-1-yl)-2,2,-dimethyl piperazine salt that is used in pharmaceuticals, specifically an agent for schizophrenia.

As a method for synthesizing optically active 6-chloro-3-phenylindanol, for example, the following methods are known.
Method i : a method of separating one enantiomer from racemic 6-chloro-3-phenylindanone using a chiral column and diastereoselectively reducing the obtained 6-chloro-3-phenylindanone (Patent Document 1)
Method ii: a method of diastereoselectively reducing racemic 6-chloro-3-phenylindanone to produce racemic 6-chloro-3-phenylindanol, and separating one optical isomer therefrom to obtain desired optically active 6-chloro-3-phenylindanol (Patent Document 2). The methods of separating one optical isomer include the following three - methods a) to c).
   a) a method of separating by using chiral chromatography
   b) a method of separating by enantioselectively acylating a hydroxyl group using an enzyme
   c) a method of separating by enantioselective deacylating after acylation of a hydroxyl group using an enzyme

### PRIOR ART DOCUMENTS

### PATENT DOCUMENTS

Patent Document 1: WO 2006/086984
Patent Document 2: WO 2005/016901

WO 01/49649 (A1) describes a process for the enantioselective preparation of tolterodine and analogues and salts thereof, comprising the steps of:
a) enantioselectively reducing the carbonyl function in a compound of formula (II), wherein R₁, R₂, and R₃ independently of each other are hydrogen, methyl, methoxy, hydroxy, hydroxymethyl, carbamoyl, sulphamoyl or halogen, to form an enantiomerically enriched compound of formula (IIIa) or (IIIb);
b) subjecting the compound of formula (IIIa) or (IIIb) to a sigmatropic rearrangement to form a corresponding enantiomerically enriched compound of formula (IVa) or (IVb);
c) subjecting the compound of formula (IVa) or (IVb) to a Baeyer-Villiger oxidation to form a corresponding enantiomerically enriched compound of formula (Va) or (Vb);
d) converting the compound of formula (Va) or (Vb) to form the corresponding enantiomerically enriched compound of tolterodine or analogue thereof; and
e) optionally converting a compound obtained in base form to a salt thereof, or converting a salt form to the free base.

### SUMMARY OF THE INVENTION

### PROBLEMS TO BE SOLVED BY THE INVENTION

However, in the above methods i) and ii), chiral resolution
is performed in order to obtain an optically active substance, thus it is difficult to say that it is an efficient production method. There is also a method that reuses a compound having a conformation different from that of a target compound by racemization, but it requires a plurality of steps for reuse, and it cannot necessarily be said to be efficient.

### SOLUTIONS TO THE PROBLEMS

The present inventors have intensively studied in view of the problems of the above-described conventional arts relating to the production of optically active aryl indanol derivatives . As a result, an enantioselective reduction reaction of a halogenated indenone derivative is utilized, which has led to the efficient production of an optically active indanol derivative. In addition, an aryl indenone derivative, which is a raw material of the enantioselective reduction reaction, is conventionally synthesized by Heck reaction using a palladium catalyst to beta-aryl enone, and the like. However, when a raw material having an electron withdrawing substituent such as 6-chloro-3-phenylindenone is used, Heck reaction is not well progressed, thus an efficient synthesis is difficult (see Organic Letter, 1999, 1, 1839). In the present application, a method that is capable of an efficient synthesis even in the case of an aryl indenone derivative having an electron withdrawing substituent has been found.

The present invention relates to a method for producing an optically active ketone, as described in claims 1 to 14.

That is, the present invention according to claim 1 relates to a method for producing an optically active ketone, comprising the step of:
stereoselectively reducing an enone compound represented by the following formula (1) by means of an enzyme source or an asymmetric metal catalyst: 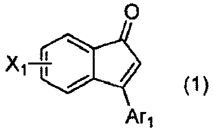 wherein Ar₁ represents a furyl group, a thienyl group or a C₆₋₂₀ aryl group, wherein these groups are optionally substituted by one, two or more substituents; and X₁ represents a halogen atom,
to produce an optically active ketone represented by the following formula (2): 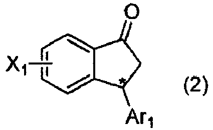 wherein, Ar₁ and X₁ represent the same as the above, and * indicates an asymmetric carbon atom.

Herein also described is a method for producing an optically active ketone, comprising the step of:
stereoselectively reducing a carbonyl group of the enone compound represented by the formula (1) to produce a compound represented by the following formula (3): 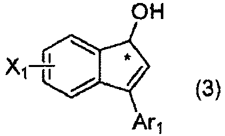 wherein, Ar₁ and X₁ represent the same as the above, and * indicates an asymmetric carbon atom,
and then rearranging the compound (3) to produce the optically active ketone represented by the formula (2).

Furthermore, according to claim 9, the present invention relates to a method for producing an optically active alcohol, comprising the step of:
stereoselectively reducing the carbonyl group of the optically active ketone represented by the formula (2) produced by the method described above,
to produce an optically active alcohol represented by the following formula (5): 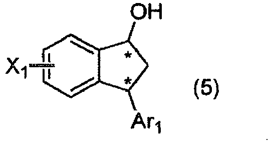 wherein, Ar₁ and X₁ represent the same as the above, and * indicates an asymmetric carbon atom.

Additionally, according to claim 17, the present invention relates to a method for producing an enone compound, comprising the steps of:
acting a halogenating agent on a ketone compound represented by the following formula (6): 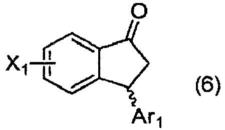 wherein, Ar₁ and X₁ represent the same as the above to produce a compound represented by the following formula (7): 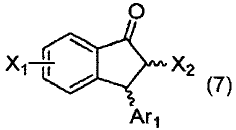 wherein, Ar₁ and X₁ represent the same as the above; and X₂ represents a halogen atom, and
reacting the compound (7) with a base, to produce a ketone compound represented by the following formula (1): 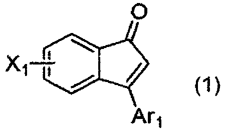 wherein, Ar₁ and X₁ represent the same as the above.

In addition, the present application describes a compound represented by the formula (1).

The present application also describes a method for producing an optically active alcohol, comprising a step of:
acting an asymmetric metal catalyst having an ability to stereoselectively reduce a carbonyl group, a hydrogen donating compound and a base on the enone compound represented by the compound (1), to produce the optically active alcohol represented by the formula (5).

Additionally, described is a method for producing a 1-piperazino-1,2-dihydroindene derivative, comprising a step of:
stereoselectively reducing the enone compound represented by the formula (1) to convert the compound (1) into the optically active ketone represented by the formula (2),
wherein, a 1-piperazino-1,2-dihydroindene derivative is represented by the following formula (8): 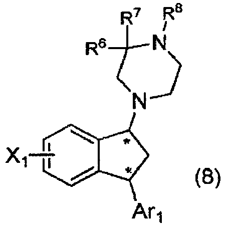 wherein, Ar₁ and X₁ represent the same as the above; R⁶, R⁷ and R⁸ are independent of each other and represent a hydrogen atom, a C₁₋₁₂ alkyl group, an alkenyl group, a C₆₋₁₄ aryl group, a C₇₋₁₅ aralkyl group, a cycloalkyl group or a cycloalkylalkyl group; R⁶ and R⁷ may be linked with each other to form a ring; R⁷ and R⁸ may be linked with each other to form a ring; and * indicates an asymmetric carbon atom.

### EFFECTS OF THE INVENTION

In the present invention, halogenated indenones are subjected to an enantioselective reduction reaction, and thus optically active compounds in which the indene or indane skeleton is maintained (optically active indanones, optically active indenols, optically active indanols, etc.) can be produced, and a target compound can be highly efficiently obtained at low costs. For example, according to the method of the present invention for producing an optically active indanol derivative from a halogenated indenone derivative, a target compound can be highly efficiently obtained at low costs . In particular, an enantioselective reduction is used, and thus productivity can be improved as compared to a conventional method. In addition, a halogenated indenone derivative is produced from racemic halogenated indanone, and thus an optically active indanol derivative can be more efficiently produced. Therefore, the present invention can be industrially suitably used.

### MODE FOR CARRYING OUT THE INVENTION

The present invention relates to a method for producing an optically active ketone, as described in claims 1 to 14.

Hereinbelow, the present invention will be described in detail.

The present invention uses halogenated indenones in an enantioselective reduction reaction. In this reduction reaction, optically active compounds in which the indene or indane skeleton is maintained (optically active indanones, optically active indenols, optically active indanols, etc.) may be produced.

Hereinbelow, examples included in this method will be described in turn.

### 1) First Example

A first example includes a method for producing an optically active ketone comprising a step of:
stereoselectively reducing an enone compound represented by the following formula (1): 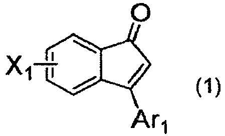 (hereinafter, referred to as an enone compound (1). It corresponds to the above-mentioned halogenated indenones) to produce an optically active ketone represented by the following formula (2): 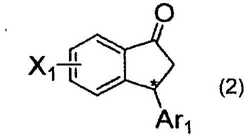 (hereinafter, referred to as an optically active ketone (2) . It corresponds to the above-mentioned optically active indanones) .

In the formula (1), Ar₁ represents
a furyl group or a thienyl group, or a C₆₋₂₀ aryl group (a phenyl group, a naphthyl group, etc.). These may have one or two or more substituents. Examples of the substituent include halogen atoms such as fluorine, chlorine, bromine, and iodine, C₁₋₂₀ alkyl groups (particularly, a methyl group, an ethyl group, a propyl group, an isopropyl group, a butyl group, etc.), C₆₋₁₀ aromatic rings (a phenyl group, etc.), a nitro group, a nitroso group, a cyano group, an amino group, a hydroxyamino group, a C₁₋₂₀ alkylamino group, a C₁₋₂₀ dialkylamino group, a C₇₋₂₀ aralkyl amino group, a C₇₋₂₀ diaralkyl amino group, a C₁₋₂₀ alkylsulfonylamino group, a sulfonate group, a sulfonamide group, an azido group, a trifluoromethyl group, a carboxyl group, a C₁₋₂₀ acyl group, a C₇₋₂₀ aroyl group, a hydroxyl group, C₁₋₂₀ alkyloxy groups (particularly, a methoxy group, etc.), a C₇₋₂₀ aralkyloxy group, a C₆₋₂₀ aryloxy group, a C₁₋₂₀ acyloxy group, a C₇₋₂₀ aroyloxy group, a C₃₋₂₀ silyloxy group, a C₁₋₂₀ alkylsulfonyloxy group, a C₁₋₂₀ alkylthio group, and the like.

Ar₁ specifically includes a phenyl group, a 4-chlorophenyl group, a 3-chlorophenyl group, a 2-chlorophenyl group, a 4-hydroxyphenyl group, a 3-hydroxyphenyl group, a 2-hydroxyphenyl group, a 4-fluorophenyl group, a 3-fluorophenyl group, a 2-fluorophenyl group, a 4-bromophenyl group, a 3-bromophenyl group, a 2-bromophenyl group, a 4-trifluoromethylphenyl group, a 3-trifluoromethylphenyl group, a 2-trifluoromethylphenyl group, a 1-naphthyl group, a 2-naphthyl group, a 4-methylphenyl group, a 3-methylphenyl group, a 2-methylphenyl group, a 4-ethylphenyl group, a 3-ethylphenyl group, a 2-ethylphenyl group, a 4-methoxyphenyl group, a 3-methoxyphenyl group, a 2-methoxyphenyl group, a 4-nitrophenyl group, a 4-phenylphenyl group, a 2,4, 6-trimethylphenyl group, a 2,4,6-triisopropylphenyl group, a thienyl group, a 2-fluorothienyl group, a 2-chlorothienyl group, a 2-bromothienyl group, a 2-iodothienyl group, a 3-fluorothienyl group, a 3-chlorothienyl group, a 3-bromothienyl group, a 3-iodothienyl group, a furyl group, a 2-fluorofuryl group, a 2-chlorofuryl group, a 2-bromofuryl group, a 2-iodofuryl group, a 3-fluorofuryl group, a 3-chlorofuryl group, a 3-bromofuryl group, a 3-iodofuryl group, a 2-methylthienyl group, a 2-ethylthienyl group,
a 2-butylthienyl group, a 3-methylthienyl group, a 3-ethylthienyl group, a 3-propylthienyl group, a 3-butylthienyl group, a 2-methylfuryl group, a 2-ethylfuryl group, a 2-propylfuryl group, a 2-butylfuryl group, a 3-methylfuryl group, a 3-ethylfuryl group, a 3-propylfuryl group, a 3-butylfuryl group, and the like. Ar₁ is preferably a phenyl group, a furyl group, and a thienyl group, and further preferably a phenyl group.

X₁ represents a halogen atom, and may be in any position on the benzene ring. X₁ specifically includes fluorine, chlorine, bromine, and iodine, and is preferably chlorine. Further preferably, X₁ binds to the 6-position of indan-1-one skeleton (in the case of the compound (5) described below, indan-1-ol skeleton), and this X₁ is chlorine.

In the formula (2), Ar₁ and X₁ represent the same as the above. * indicates an asymmetric carbon (asymmetric point, chiral center).

In the first example,
a) an enzyme source having an ability to stereoselectively reduce a carbon-carbon double bond of an enone site or
b) an asymmetric metal catalyst having an ability to stereoselectively reduce a carbon-carbon double bond of the enone site
acts on the enone compound (1) to produce the optically active ketone (2).

First, a reduction using the a) enzyme source will be described. The reaction of stereoselectively reducing a carbonyl group of the enone compound (1) and the reaction of stereoselectively reducing a carbonyl group of the optically active ketone (2) described below are also similarly practicable by properly changing the microorganism that is an origin of the enzyme source and the substrate to be used.

As the enzyme source to be used, any of those having an ability to stereoselectively reduce a carbon-carbon double bond of the enone site of the enone compound (1) to produce the optically active ketone (2) may be used.

The "enzyme source" may be not only an enzyme itself, but also a microbial cells itself that produces the enzyme, a culture broth of the microorganism, and a processed microbial cells, so long as they have an intended reduction activity. In addition, a transformant into which DNA encoding the enzyme having a reduction activity derived from the microorganism is also included.

The processed microbial cells is not particularly limited, and examples thereof include dried microbial cells obtained by dehydration with acetone or diphosphorus pentoxide, or by drying with a desiccator or a fan, surfactant processed materials, lytic enzyme processed materials, immobilized microbial cells, cell-free extracts obtained by disrupting microbial cells, and the like. Furthermore, an enzyme which catalyzes an asymmetric reduction reaction may be purified from the cultured product and used.

These may be used alone or in combination of two or more thereof. Also, these microorganisms may be immobilized by a well-known method and used.

The microorganism having an ability to stereoselectively reduce a carbon-carbon double bond of the enone site of the enone compound (1) can be found, for example, according to the method described below.

In a test tube, a liquid culture medium (5 mL, pH 7) containing glucose (40 g), an yeast extract (3 g), diammonium hydrogen phosphate (6.5 g), potassium dihydrogen phosphate (1 g), magnesium sulfate heptahydrate (0.8 g), zinc sulfate heptahydrate (60 mg), iron sulfate heptahydrate (90 mg), copper sulfate pentahydrate (5 mg), manganese sulfate tetrahydrate (10 mg), and sodium chloride (100 mg) is placed (per liter each) and sterilized, then the microorganism is aseptically inoculated and cultured under shaking at 30°C for 2 to 3 days.

Thereafter, the microbial cells are collected through centrifugation, and suspended in 1 to 5 ml of a phosphate buffer solution containing 2 to 10% of glucose. The suspension is added to a test tube in which 2.5 to 25 mg of the enone compound (1) has been previously placed, and shaken at 30°C for 2 to 3 days. At this time, the microbial cells prepared by drying, in a desiccator or with acetone, the microbial cells that are obtained through centrifugation can be also used. Furthermore, when these microorganisms or processed materials thereof are allowed to react with the enone compound (1), oxidized nicotinamide adenine dinucleotide (hereinafter, referred to as NAD⁺) and/or oxidized nicotinamide adenine dinucleotide phosphate (hereinafter, referred to as NADP⁺) may be added, and further, a glucose dehydrogenase may be added together with glucose, or a formate dehydrogenase may be added together with formic acid. In addition, an organic solvent may be allowed to coexist in the reaction system. After the conversion reaction, extraction is carried out with a suitable organic solvent, and whether or not the optically active ketone (2) is produced may be confirmed by high performance liquid chromatography or the like.

Examples of the enzyme source having an ability to stereoselectively reduce a carbon-carbon double bond of the enone site of the enone compound (1) to produce the optically active ketone (2) include an Old Yellow Enzyme and an enone reductase, and those include oxidoreductases classified into EC 1.6.99 according to enzyme classification of the International Union of Biochemistry and Molecular Biology. The oxidoreductases classified into EC 1.6.99 include oxidoreductases classified into EC 1.6.99.1: NADPH dehydrogenase, EC 1.6.99.2: NAD(P)H dehydrogenase (quinone), EC 1.6.99.3: NADH dehydrogenase, EC 1.6.99.5: NADH dehydrogenase (quinone), and EC 1.6.99.6: NADPH dehydrogenase (quinone).

The EC 1.6.99 NADPH dehydrogenase specifically includes those derived from yeasts such as genera Candida, Kluyveromyces, Saccharomyces, and Schizosaccharomyces, and those derived from bacteria such as genera Bacillus, Escherichia, Pseudomonas, Yersinia, and Zymomonas; preferably includes those derived from microorganism such as genera Saccharomyces, Kluyveromyces, Bacillus, Escherichia, Pseudomonas, Yersinia and Zymomonas; and most preferably includes Saccharomyces cerevislae, Kluyveromyces lactis, Bacillus subtilis, Escherichia coli, Pseudomonas putida, Yersinia bercovieri and Zymomonas mobilis.

Examples of the most preferable enzyme produced by the microorganism include OYE2, OYE3 (derived from Saccharomyces cerevislae, described in International Publication WO 2006/129628), KYE (derived from Kluyveromyces lactis, described in Adv. Synth. Catal., 349, 1521(2007)), YqjM (derived from Bacillus subtilis, described in J. Biol. Chem., 278, 19891(2003)), NemA (derived from Escherichia coli, described in Biol. Pharm. Bull. 20, 110 (1997)), XeaA, XenE and the like (derived from Pseudomonas putida, described in Appl. Environ. Microbiol., 74, 6703(2008)), YersER (derived from Yersinia bercovieri, described in Adv. Synth. Catal., 349, 1521 (2007)), NCR-R (derived from Zymomonas mobilis, described in Biotechnol. Bioeng., 98, 22(2007)), and the like.

Normally, these microorganisms can be obtained from stock strains that are easily available or purchased. For example, the microorganisms are available from the following culture collection.
- The Incorporated Administrative Agency, National Institute of Technology and Evaluation, Department of Biotechnology, NITE Biological Resource Center (NBRC) (2-5-8 Kazusakamatari, Kisarazu-shi, Chiba, 292-0818, Japan)
- The Incorporated Administrative Agency, RIKEN, BioResource Center, Japan Collection of Microorganisms (JCM) (2-1 Hirosawa, Wako-shi, Saitama, 351-0198, Japan)
- German Collection of Microorganisms and Cell Cultures GmbH (DSMZ) (Marschroder Weg 1b, D-38124 Brunswick, Germany)

In addition, the reductase used in the present invention (e.g., OYE2) may have the amino acid sequence (for example, an amino acid sequence of OYE2 described in J. Biol. Chem. 268, 6097-6106 (1993)), a polypeptide in which one or a plurality (for example, 40, preferably 20, more preferably 15, further preferably 10, and further preferably 5, 4, 3, or 2 or less) of amino acids are substituted, inserted, deleted, and/or added, so long as it has an intended reductase activity. Further, an additional amino acid sequence may be bonded to an amino acid sequence of the reductase used in the present invention, so long as it has an intended reductase activity. For example, a tag sequence such as histidine tag or HA tag can be added. Alternatively, the reductase may be also a fusion protein with other proteins. In addition, the reductase may be a peptide fragment, so long as it has an intended reductase activity.

In the present invention, when a transformant containing DNA encoding an enone reductase is used, the optically active ketone (2) can be more efficiently produced. Incidentally, gene manipulations such as isolation of DNA, preparation of vector and transformation, which are described in the present specification, can be performed according to the methods described in books such as Molecular Cloning 2nd Edition (Cold Spring Harbor Laboratory Press, 1989) and Current Protocols in Molecular Biology (Greene Publishing Associates and Wiley-Interscience), unless otherwise stated,

A vector used in the transformant is not particularly limited so long as it is capable of expressing a gene encoding the reductase used in the present invention in an appropriate host organism. Examples of the vector include plasmid vectors, phage vectors, cosmid vectors, and the like, and furthermore, shuttle vectors capable of undergoing genetic exchange with other host strains can be also used.

For example, in the case of Escherichia coli, such a vector normally contains regulatory factors such as a lacUV5 promoter, a trp promoter, a trc promoter, a tac promoter, a 1pp promoter, a tufB promoter, a recA promoter, and a pL promoter, and can be suitably used as an expression vector containing an expression unit operably linked to the DNA of the present invention. Examples thereof include pSTV28 (manufactured by Takara Bio Inc.), pUCNT (International Publication WO 94/03613), and the like.

The term "regulatory factor" refers to a base sequence comprising a functional promoter and an arbitrary related transcription element (e.g., enhancer, CCAAT box, TATA box, SPI site, etc.).

Also, the phrase "operably linked" means that various regulatory elements, such as promoters and enhancers which control gene expression, are linked to a gene in a state such that they are operable in host cells. It is a well known matter to those skilled in the art that the type and kind of regulatory factor vary depending on the host species.

Vectors, promoters and the like which can be used in various organisms are described in detail in "Basic Courses in Microbiology 8: Genetic Engineering)" (KYORITSU SHUPPAN CO., LTD., 1987) and the like.

The host organism to be used for the expression of each enzyme is not particularly limited so long as it is an organism capable of being transformed with an enzyme expression vector containing DNA encoding each enzyme and capable of expressing the enzyme into which DNA is introduced.

Examples of the microorganisms which can be used include bacteria whose host-vector system has been developed, such as genera Escherichia, Bacillus, Pseudomonas, Serratia, Brevibacterium, Corynebacterium, Streptococcus, and Lactobacillus; actinomycetes whose host-vector system has been developed, such as genera Rhodococcus and Streptomyces; yeasts whose host-vector system has been developed, such as genera Saccharomyces, Kluyveromyces, Schizosaccharomyces, Zygosaccharomyces, Yarrowia, Trichosporon, Rhodosporidium, Pichia, and Candida; fungi whose host-vector system has been developed, such as genera Neurospora, Aspergillus, Cephalosporium, and Trichoderma; and the like. In addition to those of the microorganisms, various host-vector systems have also been developed in plants and animals. In particular, systems expressing foreign proteins in large quantities in insects such as silkworms (Nature 315, 592-594 (1985)) or in plants such as rapeseed, maize and potato have been developed and can be suitably utilized. Among these, bacteria are preferable in terms of introduction and expression efficiency, and Escherichia coli is particularly preferable.

The vector containing DNA encoding a reductase used in the present invention can be introduced into a host microorganism by a known method. For example, when Escherichia coli is used as the host microorganism, the vector can be introduced into a host cell by using commercially available Escherichia coli HB101 (hereinafter, E. coli HB101) competent cells (manufactured by Takara Bio Inc.).

The vector containing DNA encoding an enzyme which reduces a carbon-carbon double bond of the enone site of the enone compound (1) includes pTSYE2 described in Example 5 of International Publication WO 2006/129628. In addition, an example of a transformant containing DNA encoding a 6-chloro-3-phenylindenone reductase includes E. coli HB101 (pTSYE2) obtained by transforming E. coli HB101 with vector pTSYE2. Herein, the transformant also includes E. coli HB101 (pTSYE3), E. coli HB101 (pNKYE), E. coli HB101 (pNYqjM), E. coli HB101 (pNNemA), E. coli HB101 (pNXenA), E. coli HB101 (pNXenE), E. coli HB101 (pNYersER), E. coli HB101 (pNNCR-R), and the like.

Also, in the present invention, the optically active compound can be more efficiently produced by using a transformant containing both DNA encoding an enzyme having an intended reduction activity and DNA encoding a polypeptide having an ability to reproduce a coenzyme. For example, a transformant containing both DNA encoding an enzyme which reduces a carbon-carbon double bond of the enone site of the enone compound (1) and DNA encoding a polypeptide having an ability to reproduce a coenzyme is obtained by integrating both DNA encoding an enzyme which reduces a carbon-carbon double bond of the enone site of the enone compound (1) and encoding a polypeptide having an ability to reproduce a coenzyme into one vector, and then introducing the vector into a host cell. Alternatively, such transformant and DNA may be also obtained by respectively integrating these two kinds of DNAs into two different vectors, whose incompatible groups are different, and then introducing the two vectors into one host cell. Moreover, when host cells that release an enzyme out of the cells are used, or when a liquid of crushed host cells is used in the reaction, DNA encoding an enzyme which reduces a carbon-carbon double bond of the enone site of the enone compound (1) and DNA encoding a polypeptide having an ability to reproduce a coenzyme are respectively introduced to different host cells, and the host cells may be cultured in the same culture broth or using a different culture broth.

The polypeptide having an ability to reproduce a coenzyme is preferably an oxidoreductase having an ability to convert NAD⁺ or NADP⁺ into NADH or NADPH.

Examples of such an enzyme include a hydrogenase, a formate dehydrogenase, a glucose-6-phosphate dehydrogenase, a glucose dehydrogenase, and the like. A formate dehydrogenase and a glucose dehydrogenase are suitably used.

Examples of the formate dehydrogenase include enzymes obtained from microorganisms such as genera Candida, Kloechera, Pichia, Lipomyces, Pseudomonas, Moraxella, Hyphomicrobium, Paracoccus, Thiobacillus, and Ancylobacter, and particularly, enzymes obtained from Thiobacillus sp.

Examples of the glucose dehydrogenase include enzymes obtained from microorganisms such as genera Bacillus, Lactobacillus, and Pediococcus, and particularly, enzymes obtained from Bacillus megaterium, Lactobacillus plantarum, Lactobacillus pentosus, and Pediococcus parvulus.

The enzyme is preferably an oxidoreductase that does not produce the optically active ketone (2) and an optically active alcohol (3) represented by the following formula (3): 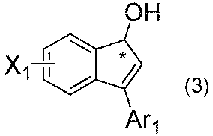 from the enone compound (1) and has an ability to convert NAD⁺ or NADP⁺ into NADH or NADPH. The enzyme is more preferably an NADP-specific enzyme. As the NADP-specific enzyme, for example, glucose dehydrogenases derived from genus Cryptococcus (JP-A-2006-262767), genus Gluconobacter (J. Bacteriol., 184, 672-678, (2002)), genus Saccharomyces (Methods Enzymol., 89, 159-163, (1982)), genus Lactobacillus, and genus Pediococcus (International Publication WO 2009/041415), and glucose-6-phosphate dehydrogenases derived from genera Cryptococcus, Aspergillus, and Pseudomonas are known (Arch. Biochem. Biophys., 228, 113-119 (1984)).

The enzyme is further preferably an enzyme derived from a lactic acid bacterium, and most preferably a glucose dehydrogenase derived from Lactobacillus pentosus, Lactobacillus plantarum or Pediococcus parvulus described in International Publication WO 2009/41415.

The reason why the oxidoreductase that does not produce the optically active ketone (2) and the optically active alcohol (3) from the enone compound (1) and has an ability to convert NAD⁺ or NADP⁺ into NADH or NADPH is preferable for the reproduction of coenzyme in the present invention will be described hereinbelow.

Among glucose dehydrogenases and formate dehydrogenases, there are enzymes that reduce the carbon-carbon double bond of the enone site or the carbonyl group of the enone compound (1) and produce the optically active ketone (2) and the optically active alcohol (3). When these enzymes are used for the reproduction of coenzyme, there is a possibility to cause low yield and low optical purity due to the production of unintended compounds.

For example, a glucose dehydrogenase derived from Bacillus megaterium described in International Publication WO 2006/033333 reduces the carbonyl group of the enone compound
(1) to produce an (R) -enantiomer of the optically active alcohol (3) (see Example 4 of the present application). Furthermore, the (R)-enantiomer of the optically active alcohol (3) may be converted into an (R) -enantiomer of the optically active ketone
(2) by the rearrangement reaction. Specifically, in the case of producing an (S) -enantiomer of the optically active ketone (2), when a glucose dehydrogenase derived from Bacillus megaterium is used for the reproduction of coenzyme, low yield due to byproduct of the (R) -enantiomer of the optically active alcohol (3) and low optical purity of the (S)-enantiomer of the optically active ketone (2) due to byproduct of the (R)-enantiomer of the optically active ketone (2) are caused. In addition, in the case of producing an (S) -enantiomer of the optically active alcohol (3), when a glucose dehydrogenase derived from Bacillus megaterium is used for the reproduction of coenzyme, low optical purity of the (S)-enantiomer of the optically active alcohol (3) due to byproduct of the (R)-enantiomer of the optically active alcohol (3) is caused. As described above, the glucose dehydrogenase derived from Bacillus megaterium is not suitable for the reproduction of coenzyme in the case of producing the (S)-enantiomer of the optically active ketone (2) or the (S)-enantiomer of the optically active alcohol (3).

On the other hand, in the case of a glucose dehydrogenases specific to NADP, which are derived from a lactic acid bacterium such as Lactobacillus pentosus, Lactobacillus plantarum, Pediococcus parvulus and the like described in International Publication WO 2009/041415, the optically active ketone (2) and the optically active alcohol (3) are not produced from the enone compound (1), and thus it is preferable to use the above enzyme for the reproduction of coenzyme in the reduction reaction of the enone compound (1) using an enzyme source (see Examples 7 to 17 of the present application).

For culturing the microorganism used as an enzyme source, any culture medium containing a nutrient source which the microorganism can assimilate can be used in common. For example, common culture media prepared by properly mixing and blending carbon sources such as saccharides including a glucose, a sucrose, a maltose, and the like, organic acids including lactic acid, acetic acid, citric acid, propionic acid, and the like, alcohols including ethanol, glycerin, and the like, hydrocarbons including paraffin, and the like, fats and oils including soybean oil, rape oil, and the like, and mixtures of the above substances; nitrogen sources such as ammonium sulfate, ammonium phosphate, urea, yeast extract, meat extract, peptone and corn steep liquor; furthermore, nutrient sources such as other inorganic salts and vitamins can be used. These media may be properly selected depending on the kind of the microorganism to be used. In addition, in order to induce an intended reductase, it is preferable to add various enone compounds to the medium since excellent results can be achieved. For example, the enone compound (1) may be added to the medium by 0.01 to 50% (W/V).

The microorganism can be cultured under general conditions. For example, the microorganism is preferably cultured at a pH of 4.0 to 9.5 in a temperature range of 20°C to 45°C for 10 to 96 hours in an aerobic condition.

Next, the reduction reaction of the enone compound (1) using an enzyme source will be described.

In the reduction reaction using an enzyme source, an appropriate solvent, and the enone compound (1) which is a substrate, the above microorganism, a culture thereof, a processed material thereof or the like are mixed, and the mixture is stirred and shaken or allowed to stand still under pH adjustment.

As the reaction solvent, an aqueous medium such as water or a buffer solution is normally used. The buffer solution includes a potassium phosphate buffer solution and a tris(hydroxymethyl)aminomethane-hydrochloride buffer solution.

As the enzyme source, a culture broth containing microbial cells of the above microorganism is normally used as it is to the reaction. The culture broth may be also concentrated and used. In addition, when the component in the culture broth has an adverse effect on the reaction, microbial cells or processed microbial cells obtained by treating the culture broth through centrifugation or the like may be suitably used.

The enone compound (1), which is a substrate, may be added all at once in an initial stage of the reaction or may be added sequentially separately along with the proceeding of the reaction.

The temperature of the reaction is normally set to 10 to 60°C and preferably 20 to 40°C.

The pH of the reaction is in a range of 2.5 to 9 and preferably 5 to 9.

The amount of the microorganism in the reaction mixture may be properly determined depending on the ability to reduce these substrates, and is preferably 0.01 to 50% (W/V) and more preferably 0.1 to 10% (W/V).

The concentration of the substrate in the reaction mixture is preferably 0.01 to 50% (W/V) and more preferably 0.1 to 30% (W/V).

The reaction is normally carried out while shaking or ventilating and stirring.

The reaction time is properly determined depending on the concentration of the substrate, the amount of the microorganism, and other reaction conditions. In common, it is preferable to set each condition so as to complete the reaction in 2 to 168 hours.

In order to promote the reduction reaction, an energy source such as glucose, ethanol or isopropanol is preferably added to the reaction mixture at a ratio of 0.5 to 30% (W/V).

Generally, a coenzyme such as reduced nicotinamide-adenine dinucleotide (hereinafter abbreviated as NADH) or reduced nicotinamide-adenine dinucleotide phosphoric acid (hereinafter abbreviated as NADPH), which is required in a reduction reaction carried out by a biological method, can be also added to promote the reaction. In this case, the coenzyme is normally added directly to the reaction mixture.

In addition, in order to promote the reduction reaction, it is preferable to carry out the reaction in coexistence with an enzyme to reduce NAD⁺ and/or NADP⁺ to its reduced type and a substrate for the reduction. For example, a glucose dehydrogenase as an enzyme to reduce NAD⁺ and/or NADP⁺ to its reduced type and a glucose as a substrate for the reduction may be respectively coexisted, or a formate dehydrogenase as an enzyme to reduce NAD⁺ and/or NADP⁺ to its reduced type and formic acid as a substrate for the reduction may be respectively coexisted.

Moreover, an addition of a surfactant such as Triton (manufactured by NACALAI TESQUE, INC.), Span (manufactured by KANTO CHEMICAL CO., INC.) or Tween (manufactured by NACALAI TESQUE, INC.) to the reaction mixture is also effective.

Furthermore, for the purpose of avoiding inhibition of the reaction by a substrate and/or an alcohol body, which is a product of the reduction reaction, a water-insoluble organic solvent such as ethyl acetate, butyl acetate, isopropyl ether, toluene or hexane may be added to the reaction mixture.

Also, for the purpose of increasing the solubility of the substrate, a water-soluble organic solvent such as methanol, ethanol, acetone, tetrahydrofuran or dimethyl sulfoxide can be also added.

A method of taking out the optically active ketone (2) produced by the reduction reaction is not particularly limited. The optically active ketone (2) with high purity can be easily obtained by extraction in a direct manner or after separation of microbial cells or the like from the reaction mixture with a solvent such as ethyl acetate, toluene, t-butyl methyl ether, hexane, n-butanol or dichloromethane, and after dehydration, purification of the obtained substance with distillation, silica gel column chromatography or the like.

Next, b) a reduction reaction using an asymmetric metal catalyst will be described.

The reduction reaction is carried out by acting a hydrogen donating compound on a substrate in the presence of an asymmetric metal catalyst. The asymmetric metal catalyst to be used includes metal catalysts containing Ru, Ni, Rh, Pt, Pd, Ir, or Cu, and is preferably, Ru, Rh, Ir, or Cu. The asymmetric metal catalyst may be a metal catalyst including a transition metal, and may be a catalyst other than the above examples (metal catalysts containing Zr, Ti, Cr, Co, Zn, Mn, Fe, Yb, or La).

The asymmetric metal catalyst is preferably a complex.

A chiral ligand in the metal complex is not particularly limited, and examples thereof include phosphine ligands such as (R)-BINAP, (S)-BINAP, (R)-tolBINAP, (S)-tolBINAP, (R)-SEGPHOS, (S)-SEGPHOS, (R)-JOSIPHOS, (S)-JOSIPHOS, (R)-DIOP, and (S)-DIOP, diamine ligands such as (S,S)-N-para-toluenesulfonyl-1,2-diphenylethylenediamine and (R,R)-N-para-toluenesulfonyl-1,2-diphenylethylenediamine.

In order to stereoselectively reduce a carbon-carbon double bond of the enone site, for example, combinations of an asymmetric metal catalyst and a phosphine ligand such as Rh-BINAP, Ir-BINAP, Ru-BINAP, Pd-BINAP, Cu-BINAP, Rh-SEGPHOS, Ir-SEGPHOS, Ru-SEGPHOS, Pd-SEGPHOS, and Cu-SEGPHOS are preferable.

In the present step, the amount of the asymmetric metal catalyst to be used is not particularly limited, but the amount is normally 0.00001 to 1 equivalent, preferably 0.0001 to 0.1 equivalents, and more preferably 0.0001 to 0.01 equivalents to the ketone represented by the formula (1).

In addition, the amount of the ligand to be used in the present step is not particularly limited, but the amount is normally 0.1 to 5 equivalents, preferably 0.5 equivalents to 3 equivalents, and more preferably 1 equivalent to 2 equivalents to the metal catalyst.

Examples of the hydrogen donating compound to be used include, but are not particularly limited to, hydrogen, alcohols, formic acid, formic acid salts, and polymethylhydrosiloxane.

Specific examples of the alcohols include, but are not limited to, methanol, ethanol, n-propanol, isopropanol, and the like. The alcohol is preferably isopropanol.

The formic acid salts include sodium formate and ammonium formate.

The hydrogen donating compound is preferably hydrogen, further preferably hydrogen under a high-pressure atmosphere of 3 atm or more, and particularly preferably hydrogen under a high-pressure atmosphere of 10 atm or more.

In the present step, the amount of the hydrogen donating compound to be used is not particularly limited, but the amount is normally 1 to 100 equivalents and preferably 1 to 10 equivalents to the compound represented by the formula (1).

Also, in the present step, a base may be used or may not be used, but it is desirable to use a base since the reaction can be accelerated.

Examples of the base include amine bases such as triethylamine, N,N-diisopropylethylamine, and pyridine, and inorganic bases such as potassium carbonate, sodium carbonate, and lithium carbonate.

The amount of the base to be used in the present step is not particularly limited, but in the case of, triethylamine, for example, the amount is normally 0.01 to 100 equivalents, preferably 0.1 to 10 equivalents, and more preferably 1 to 4 equivalents to the enone compound (1).

In the reaction, a solvent may be used or may not be used.

The solvent may be any of a protic solvent and an aprotic solvent. Examples of the aprotic solvent that can be used include hydrocarbon solvents such as toluene, n-hexane, and cyclohexane; ether solvents such as diethyl ether, tetrahydrofuran, 1,4-dioxane, methyl t-butyl ether, dimethoxyethane, and ethylene glycol dimethyl ether; halogenated solvents such as methylene chloride, 1,2-dichloroethane, and chlorobenzene; nitrogen-containing solvents such as acetonitrile, acetamide, dimethylformamide, and dimethylacetamide; and the like. The protic solvent may be served as the hydrogen donating compound, and alcohol solvents such as methanol, ethanol, isopropanol, and t-butanol, and the like can be used as the solvent. These solvents may be used alone or used in combination of two or more thereof. It is preferred that no solvent be used or that an aprotic solvent (particularly, tetrahydrofuran, dimethylformamide) be used.

The concentration of the substrate in the reaction mixture is preferably 1 to 50% (W/V) and more preferably 5 to 20% (W/V) .

The reaction temperature is preferably -40°C to 160°C, more preferably -20°C to 100°C, and particularly preferably 0 to 60°C.

Also, the reaction time is about 10 hours or more, and preferably 20 hours or more.

### 2) Second Example (not within the claims)

In a second example, the carbonyl group of the enone compound represented by the formula (1) (corresponding to halogenated indenones) is stereoselectively reduced to produce a compound represented by the formula (3) (referred to as an optically active alcohol corresponding to the optically active indenols). It is preferred that this optically active alcohol (3) be then rearranged to produce the optically active ketone represented by the formula (2) (corresponding to an optically active indanone).

In the formula (3), Ar₁ and X₁ represent the same as the formula (1). * indicates an asymmetric carbon atom.

First, a step of obtaining the optically active alcohol (3) from the enone compound (1) will be described.

In the present step,
a) an enzyme source having an ability to stereoselectively reduce a carbonyl group, or
b) an asymmetric metal catalyst having an ability to stereoselectively reduce a carbonyl group is allowed to act to produce the optically active alcohol (3) .

First, a) a reduction reaction using an enzyme source will be described.

As the enzyme source to be used, any of those having an ability to stereoselectively reduce a carbonyl group of the enone compound (1) to produce the optically active alcohol (3) may be used.

The enzyme source is preferably an enzyme source derived from the microorganisms selected from the group consisting of genera Candida, Ogataea, Bacillus and Brevundimonas, and further preferably an enzyme source derived from the microorganisms selected from the group consisting of Candida magnoliae, Ogataea minuta var. minuta, Bacillus megaterium and Brevundimonas diminuta.

Enzymes producing an (S)-enantiomer of the optically active alcohol (3) are most preferably a carbonyl reductase derived from the Candida magnoliae IFO0705 strain described in JP Patent 4510351, a carbonyl reductase derived from the Ogataea minuta var. minuta NBRC0975 strain described in International Publication WO 2006/013801, and a carbonyl reductase derived from the Brevundimonas diminuta NBRC12697 strain described in International Publication WO 2007-114217.

Enzymes producing (R)-6-chloro-3-phenylindenol are most preferably a carbonyl reductase derived from the Ogataea minuta var. minuta NBRC0975 strain described in International Publication WO 2006/013801 and a glucose dehydrogenase derived from the Bacillus megaterium IAM1030 strain described in International Publication WO 2006/033333.

The reduction reaction can be carried out in the same manner as in the first example that obtains the optically active ketone (2) from the enone compound (1), except for using the enzyme source described above.

Next, b) a reduction reaction using an asymmetric metal catalyst will be described.

The reduction reaction is carried out by acting a hydrogen donating compound on a substrate in the presence of an asymmetric metal catalyst.

The asymmetric metal catalyst to be used includes the asymmetric metal catalysts and the ligands of the metal complexes described in the first example.

A combination of an asymmetric metal catalyst and a diamine ligand is preferable for stereoselectively reducing a carbonyl group.

In particular, the asymmetric metal catalyst preferably includes a compound (optically active diamine complex) represented by the following formula (4): 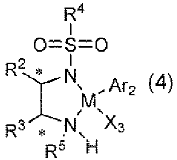 from the viewpoint of selectively reducing not the carbon-carbon double bond of an enone site of the enone compound (1) but a carbonyl group.

In the formula (4), R² and R³ represent a C₁₋₂₀ alkyl group or a C₆₋₁₄ aryl group. These may have one or two or more substituents, and the detail thereof is the same as R⁴ described below.

In addition, R² and R³ may be the same or different from each other, or further may be linked with each other to form a ring. When a ring is formed, R² and R³ represent one alkylene group by linking together.

R² and R³ preferably include a methyl group, an ethyl group, an isopropyl group, a tert-butyl group, a n-octyl group, a hydroxymethyl group, a chloromethyl group, a phenyl group, a p-hydroxyphenyl group, a benzyl group, an o, m, p-tolyl group, an o, m, p-anisyl group, a p-chlorobenzyl group, a naphthyl group, a tetramethylene group, and the like. R² and R³ are further preferably both aryl groups, or R² and R³ are a tetramethylene group by linking together to form a ring, and R² and R³ are particularly preferably both phenyl groups.

In the formula (4), R⁴ represents a C₁₋₂₀ alkyl group (including a cycloalkyl group) or a C₆₋₁₄ aryl group. These may have one or two or more substituents (nitro groups; hydroxy groups; halogen atoms such as a fluoro group, a chloro group, a bromo group, and an iodo group; haloalkyl groups; alkoxy groups; alkyl groups, aryl groups, etc.), and may form, for example, a C₇₋₁₅ aralkyl group. R⁴ that may have a substituent includes a phenyl group, a 4-chlorophenyl group, a 3-chlorophenyl group, a 2-chlorophenyl group, a 4-fluorophenyl group, a 3-fluorophenyl group, a 2-fluorophenyl group, a 4-bromophenyl group, a 3-bromophenyl group, a 2-bromophenyl group, a 4-trifluoromethylphenyl group, a 3-trifluoromethylphenyl group, a 2-trifluoromethylphenyl group, a 1-naphthyl group, a 2-naphthyl group, a 4-ethylphenyl group, a 3-ethylphenyl group, a 2-ethylphenyl group, a 4-methoxyphenyl group, a 3-methoxyphenyl group, a 2-methoxyphenyl group, a 4-phenylphenyl group, a methyl group, an ethyl group, a n-propyl group, an isopropyl group, a n-butyl group, an isobutyl group, a tert-butyl group, a n-octyl group, a hydroxymethyl group, a chloromethyl group, a trifluoromethyl group, a benzyl group, a methylcyclopropyl group, a 4-bromobutyl group, a 3-bromobutyl group, a 2-bromobutyl group, a 5-bromopentyl group, a 4-bromopentyl group, a 3-bromopentyl group, a 2-bromopentyl group, a 2-phenylethyl group, a 1-phenylethyl group, a 3-phenylbutyl group, a 2-phenylbutyl group, a 1-phenylbutyl group, a p-hydroxyphenyl group, an o, m, p-nitrophenyl group, an o, m, p-tolyl group, a p-chlorobenzyl group, a 2,4,6-trimethylphenyl group, a 2,4,6-triisopropylphenyl group, a 2,4,6-trimethoxyphenyl group, and a 2,4,6-trichlorophenyl group.

R⁴ preferably includes a methyl group, an ethyl group, an isopropyl group, a tert-butyl group, a n-octyl group, a hydroxymethyl group, a chloromethyl group, a trifluoromethyl group, a benzyl group, a phenyl group, a p-hydroxyphenyl group, an o, m, p-nitrophenyl group, an o, m, p-tolyl group, an o, m, p-trifluoromethylphenyl group, a p-chlorobenzyl group, a 2,4, 6-trimethylphenyl group, a 2,4,6-triisopropylphenyl group, a 6-trimethoxyphenyl group, a naphthyl group, a 2,4,6-trichlorophenyl group, and the like. R⁴ is further preferably a methyl group, a trifluoromethyl group, a phenyl group, a p-tolyl group, a p-trifluoromethylphenyl group, or a 2,4,6-trichlorophenyl group. R⁴ is particularly preferably a p-tolyl group.

In the formula (4), R⁵ represents a C₁₋₂₀ alkyl group, a C₆₋₁₄ aryl group, or a hydrogen atom.

R⁵ preferably includes a hydrogen atom, a methyl group, an ethyl group, an isopropyl group, a tert-butyl group, a n-octyl group, a hydroxymethyl group, a chloromethyl group, a phenyl group, a p-hydroxyphenyl group, a benzyl group, a p-chlorobenzyl group, a naphthyl group, and the like. R⁵ is further preferably a hydrogen atom or a methyl group. R⁵ is particularly preferably a hydrogen atom.

In the formula (4), Ar₂ represents an aromatic compound. Ar₂ specifically includes benzene, toluene, xylene, mesitylene, hexamethylbenzene, ethylbenzene, tert-butylbenzene, p-cymene, cumene, pentamethylcyclopentadienyl, and the like. Ar₂ is not limited to these compounds. Ar₂ is preferably p-cymene, benzene, mesitylene, or pentamethylcyclopentadienyl. Ar₂ is further preferably p-cymene.

In the formula (4), M represents a transition metal. M specifically includes Pd, Rh, Ru, Ir, Pt, Zr, Ti, Cr, Co, Cu, Ni, Zn, Mn, Fe, Yb, La, and the like. M is not limited to these metals. M is preferably Ru, Rh, Ir or Cu. M is further preferably Ru.

In the formula (4), X₃ represents a halogen atom. X₃ specifically includes fluorine, chlorine, bromine, iodine, and the like. X₃ is preferably chlorine.

In the formula (4), * indicates an asymmetric carbon atom. The configuration of the asymmetric carbon atom may be either (R) or (S) .

In an example of particularly preferable formula (4), R² and R³ are both aryl groups, or R² and R³ are a tetramethylene group by linking together to form a ring. In an example of preferable formula (4), both asymmetric carbon atoms are in the (R) configuration, or both asymmetric carbon atoms are in the (S) configuration from the viewpoint of configuration.

In the present step, the amount of the asymmetric metal catalyst to be used is not particularly limited, but the amount is usually 0.00001 to 1 equivalent, preferably 0.0001 to 0.1 equivalents, and more preferably 0.0001 to 0.01 equivalents to the enone compound (1).

The reaction conditions are basically the same as the case where an asymmetric metal catalyst is allowed to act in the first example, and hydrogen transfer reduction is performed in the presence of a hydrogen donating compound. Here, preferable hydrogen donating compound includes formic acid, formic acid salts, and alcohols, and particularly preferably includes formic acid. In addition, a reduction reaction of carbonyl can be carried out at ordinary pressure by a combination of the asymmetric metal catalyst represented by the formula (4) and formic acid. Here, a base may be used in metal catalyst reduction as described above. When a base is present, rearrangement of the obtained optically active alcohol (3) into the optically active ketone (2) is easily advanced.

Next, a step of producing the optically active ketone (2) from the optically active alcohol (3) will be described.

In the present step, a rearrangement reaction is carried out by acting a base on the optically active alcohol (3) to produce the optically active ketone (2).

In the present reaction, a solvent may be used or may not be used. Even when a solvent is used, the solvent is not particularly limited, and for example, hydrocarbon solvents such as toluene, n-hexane, and cyclohexane; ether solvents such as diethyl ether, tetrahydrofuran, 1,4-dioxane, methyl t-butyl ether, dimethoxyethane, and ethylene glycol dimethyl ether; halogenated solvents such as methylene chloride, 1,2-dichloroethane, and chlorobenzene; nitrogen-containing solvents such as acetonitrile, acetamide, dimethylformamide, and dimethylacetamide; alcohol solvents such as methanol, ethanol, isopropanol, and t-butanol; and the like can be used. These solvents may be used alone or used in combination of two or more thereof. The solvent is preferably tetrahydrofuran. The concentration of the substrate in the reaction mixture is preferably 1 to 50% (W/V) and more preferably 5 to 20% (W/V) .

Examples of the base used in the present reaction include amine bases such as triethylamine, N,N-diisopropylethylamine, pyridine, diazabicycloundecene (DBU), and 1,4-diazabicyclo[2.2.2]octane (DABCO); and inorganic bases such as potassium carbonate, sodium carbonate, lithium carbonate, potassium phosphate, sodium phosphate, potassium hydrogenphosphate, sodium hydrogenphosphate, potassium dihydrogenphosphate, and sodium dihydrogenphosphate. The base is preferably triethylamine and DABCO and further preferably DABCO.

The amount of the base to be used is not particularly limited, but the base is used in an amount of normally 0.1 to 5 equivalents, preferably 0.5 to 5 equivalents, and more preferably from 1 to 2 equivalents to the optically active alcohol (3). Here, the amount of the hydrogen donating compound may be the same. It is also possible to use the base or hydrogen donating compound in an amount exceeding these ranges, and when the amount of an agent (base and/or hydrogen donating compound) is in a large amount, the produced optically active ketone (2) is further reduced, and optically active alcohol (5) is produced by the reaction of the third example described below.

The reaction temperature is preferably -40°C to 160°C, more preferably -20°C to 100°C, and particularly preferably 0 to 60°C.

Also, the reaction time is about 0.5 hours or more, and preferably 5 hours or more.

The rearrangement reaction may be carried out after isolating the optically active alcohol (3), but by using a base in the reduction reaction, the reaction can be sequentially carried out (i.e., one pot) after the reduction reaction from the enone compound (1) to the optically active alcohol (3) without isolating the compound (3).

### 3) Third Example

The optically active ketone (2) obtained in the first example and the second example (optically active indanones) can be reduced at its carbonyl group to produce optically active indanols. In this example, specifically, the carbonyl group of the optically active ketone (2) is stereoselectively reduced to produce an optically active alcohol represented by the following formula (5): 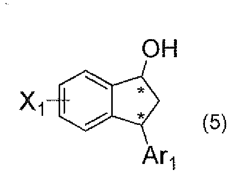 (hereinafter, optically active alcohol (5)).

In the formula (5), Ar₁ and X₁ represent the same as the above. * indicates an asymmetric carbon atom.

In the present step, the optically active alcohol (5) can be produced from the optically active ketone (2), according to any of the following methods a) to c):
a) the carbonyl group is diastereoselectively reduced using a hydride reducing agent;
b) the carbonyl group is reduced by acting an enzyme source having an ability to stereoselectively (diastereoselectively) reduce a carbonyl group; and
c) the carbonyl group is stereoselectively reduced by using an asymmetric metal catalyst having an ability to stereoselectively (diastereoselectively) reduce a carbonyl group.

First, a step a) of diastereoselectively reducing the carbonyl group using a hydride reducing agent will be described.

The hydride reducing agent is not particularly limited, and specific examples thereof include boron hydride compounds such as diborane, borane-diethylether, borane dimethylsulfide, borane-pyridine, and borane-picoline; boron hydride metal compounds such as lithium borohydride, sodium borohydride, potassium borohydride, zinc borohydride, lithium triethylborohydride, sodium triethylborohydride, potassium triethylborohydride, and sodium cyanoborohydride; aluminum hydride metal compounds such as lithium aluminum hydride, sodium aluminum hydride, and diisobutylaluminum hydride; and the like. Among these, the hydride reducing agent is preferably lithium borohydride, sodium borohydride, potassium borohydride, lithium aluminum hydride, or sodium aluminum hydride, and further preferably, sodium borohydride, potassium borohydride, or lithium aluminum hydride, and particularly preferably sodium borohydride or lithium aluminum hydride.

The amount of the reducing agent is normally 0.25 equivalents to 10 equivalents, preferably 0.25 equivalents to 5 equivalents, and further preferably 0.25 equivalents to 2 equivalents to the optically active ketone (2).

A solvent is used in the present reaction. The solvent to be used is not particularly limited, and for example, hydrocarbon solvents such as toluene, n-hexane, and cyclohexane; ether solvents such as diethyl ether, tetrahydrofuran, 1,4-dioxane, methyl t-butyl ether, dimethoxyethane, and ethylene glycol dimethyl ether; halogenated solvents such as methylene chloride, 1,2-dichloroethane, and chlorobenzene; nitrogen-containing solvents such as acetonitrile, acetamide, dimethylformamide, and dimethylacetamide; alcohol solvents such as methanol, ethanol, isopropanol, and t-butanol; and the like can be used. These solvents may be used alone or used in combination of two or more thereof . The solvent is preferably methanol or ethanol.

The concentration of the substrate in the reaction mixture is preferably 1 to 50% (W/V) and more preferably 5 to 20% (W/V).

The reaction temperature is preferably -40°C to 100°C, more preferably -20°C to 80°C, and particularly preferably 0 to 40°C.

Next, a step b) of reducing the carbonyl group by acting an enzyme source having an ability to stereoselectively reduce a carbonyl group will be described.

As the enzyme source, any of those having an ability to stereoselectively reduce the carbonyl group of the optically active ketone (2) to produce the optically active alcohol (5) may be used.

The enzyme source is preferably an enzyme source derived from the microorganisms selected from the group consisting of genera Candida, Ogataea, Saccharomyces, Brevundimonas, Devosia, Paenibacillus, and Pseudomonas, and further preferably, an enzyme source derived from the microorganisms selected from the group consisting of Candida magnoliae, Candida maltosa, Candida maris, Ogataea minuta var. minuta, Saccharomyces cerevisiae, Brevundimonas diminuta, Devosia riboflavina, Paenibacillus alvei, and Pseudomonas stutzeri. For example, enzymes producing (S, S) -6-chloro-3-phenylindanol are most preferably a carbonyl reductase derived from the Candida magnoliae IFO0705 strain described in JP Patent 4510351, a carbonyl reductase derived from the Candida magnoliae NBRC0661 strain described in International Publication WO 2006/046455, an alcohol dehydrogenase derived from the Candida maltosa IFO1977 strain described in International Publication WO 2008/066018, a carbonyl reductase derived from the Candida maris IFO10003 strain described in International Publication WO 2001/05996, a carbonyl reductase derived from the Ogataea minuta var. minuta NBRC0975 strain described in International Publication WO 2006/013801, a carbonyl reductase derived from the Saccharomyces cerevisiae S288C (ATCC26108) strain described in JP-A-2010-130912, a carbonyl reductase derived from the Brevundimonas diminuta NBRC12697 strain described in International Publication WO 2007/114217, a carbonyl reductase derived from the Devosia riboflavina IFO13584 strain described in JP Patent 4414337, a carbonyl reductase derived from the Paenibacillus alvei NBRC3343 strain described in International Publication WO 2007/099764, and a carbonyl reductase derived from the Pseudomonas stutzeri NBRC13596 strain described in International Publication WO 2007/099994.

The reduction reaction can be carried out in the same manner as in the first example described above.

Next, (c) a reduction reaction using an asymmetric metal catalyst will be described.

The reduction reaction is carried out by acting a hydrogen donating compound on a substrate in the presence of an asymmetric metal catalyst.

The asymmetric metal catalyst to be used includes the asymmetric metal catalysts and the ligand of the metal complex used in the second example.

A preferred combination of an asymmetric metal catalyst and a ligand includes those described in the second example.

Among them, the asymmetric metal catalyst includes the compound represented by the formula (4) described as a preferred asymmetric metal catalyst when stereoselectively reducing the carbonyl group of the enone compound. The reaction conditions and the amount of an agent are the same.

The present reaction may be carried out after isolating the optically active ketone (2) or can be also continuously carried out with the step of producing the optically active ketone (2) from the enone compound (1) (for example, a step of reducing the enone compound (1) to obtain the optically active alcohol (3) and rearranging the alcohol to produce the optically active ketone (2)). By shortening the isolation procedure, it can be said as a very efficient production method since a two-stage reduction reaction can be continuously carried out.

As described above, the halogenated indenones are useful for producing an optically active compound in which the indene or indane skeleton is maintained (optically active indanones, optically active indenols, optically active indanols, etc.). It is possible to efficiently synthesize the halogenated indenones, for example, as described below.

Specifically, a ketone compound represented by the following formula (6) is selected as a starting material, and subsequently, the compound represented by the formula (1) can be produced by acting a halogenating agent on the ketone compound represented by the formula (6): 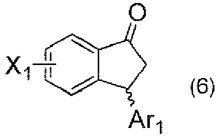 to produce a compound represented by the following formula (7) : 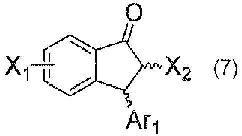 and reacting the compound (7) with a base.

In the formulae (6) and (7), Ar₁ and X₁ represent the same as the above.

In the formula (7), X₂ represents a halogen atom. Specifically, X₂ includes fluorine, chlorine, bromine, iodine, and the like. X₂ is preferably bromine.

First, a step of reacting the compound represented by the formula (6) with a halogenating agent to produce the compound represented by the formula (7) will be described.

Examples of the halogenating agent to be used include bromine, iodine, N-bromosuccinimide, N-chlorosuccinimide, N-iodosuccinimide, SO₂Cl₂, and the like. The halogenating agent is preferably N-bromosuccinimide or bromine, and further preferably bromine.

The amount of the hydrogenating agent is normally 0.8 to 2 equivalents, preferably 0.9 to 1.5 equivalents, and more preferably 0.9 to 1.3 equivalents to the compound represented by the formula (6).

A solvent may be used or may not be used. Examples of the solvent that can be used include hydrocarbon solvents such as toluene, n-hexane, and cyclohexane; ether solvents such as diethyl ether, tetrahydrofuran, 1,4-dioxane, methyl t-butyl ether, dimethoxyethane, and ethylene glycol dimethyl ether; halogenated solvents such as methylene chloride, 1,2-dichloroethane, and chlorobenzene; nitrogen-containing solvents such as acetonitrile, acetamide, dimethylformamide, and dimethylacetamide; alcohol solvents such as methanol, ethanol, isopropanol, and t-butanol; and the like. These solvents may be used alone or used in combination of two or more thereof. It is preferred that no solvent be used or that halogenated solvents such as methylene chloride, 1,2-dichloroethane, and chlorobenzene; ether solvents such as diethyl ether, tetrahydrofuran, 1,4-dioxane, methyl t-butyl ether, dimethoxyethane, and ethylene glycol dimethyl ether be used. The solvent is particularly preferably methylene chloride, methyl t-butyl ether, or diethyl ether.

The concentration of the substrate in the reaction mixture is preferably 1 to 50% (W/V) and more preferably 5 to 20% (W/V) .

The reaction temperature is preferably -40°C to 80°C, more preferably -20°C to 60°C, and particularly preferably 0 to 40°C.

In particular, in the case of using bromine as the halogenating agent, setting the amount of bromine to be used to 0.9 to 1.3 equivalents, controlling the reaction temperature to 40°C or less, and using methylene chloride or methyl t-butyl ether as the solvent, dibromination that is a main side reaction in the present reaction can be markedly suppressed, and the compound represented by the formula (7) can be produced very efficiently.

Next, a step of producing the enone compound (1) from the compound represented by the formula (7) will be described.

In the present step, a base is allowed to act on the compound represented by the formula (7) to produce the enone compound (1) .

Examples of the base to be used in the present step include amine bases such as triethylamine, N,N-diisopropylethylamine, and pyridine; and inorganic bases such as carbonates including potassium carbonate, sodium carbonate, lithium carbonate and the like; and hydroxides including lithium hydroxide, sodium hydroxide, potassium hydroxide and the like. The base is preferably triethylamine or potassium carbonate.

The amount of the base to be used is normally 0.8 to 3 equivalents, preferably 0.9 to 2 equivalents, and more preferably 1 to 1.5 equivalents to the compound represented by the formula (7).

In the present reaction, a solvent may be used or may not be used.

Examples of the solvent that can be used include hydrocarbon solvents such as toluene, n-hexane, and cyclohexane; ether solvents such as diethyl ether, tetrahydrofuran, 1,4-dioxane, methyl t-butyl ether, dimethoxyethane, and ethylene glycol dimethyl ether; halogenated solvents such as methylene chloride, 1,2-dichloroethane, and chlorobenzene; nitrogen-containing solvents such as acetonitrile, acetamide, dimethylformamide, and dimethylacetamide; alcohol solvents such as methanol, ethanol, isopropanol, and t-butanol; ketone solvents such as acetone and cyclohexanone; and the like. The solvent is preferably toluene, acetone, or N,N-dimethylformamide (DMF).

The concentration of the substrate in the reaction mixture is preferably 1 to 50% (W/V) and more preferably 5 to 20% (W/V).

The reaction temperature is preferably -40°C to 80°C, more preferably -20°C to 60°C, and particularly preferably 0 to 40°C.

The present step may be carried out after isolating the compound represented by the formula (7) or may be also continuously carried out (without isolation) with the step of halogenating the compound represented by the formula (6) without isolation.

The compound represented by general formula (1), preferably a compound wherein Ar₁ is a chlorophenyl group, a thienyl group, a furyl group or a phenyl group, a compound represented by the formula (1) wherein X₁ is a chloro group, and particularly a compound represented by the formula (1) wherein Ar₁ is a phenyl group, and X₁ is a chloro group bonded to the 6-position (the 6-position of inden-1-one skeleton) have never been synthesized so far. These are the compounds newly synthesized by the present inventors, and are useful intermediates for producing a trans-4-((1R,3S)-6-chloro-3-phenylindan-1-yl)-2,2-dimethylp iperazine salt that is used in an agent for schizophrenia.

Herein, an asymmetric metal catalyst, a hydrogen donating compound and a base are allowed to act on the enone compound (1) to produce the compound represented by the formula (2) or (3). Then, the reduction reaction is continuously carried out without post-treatment, and thus it is possible to produce the optically active alcohol (5) without isolating the compound represented by the formula (2) or (3).

According to the above method, the optically active ketone compound (2) can be efficiently produced. Therefore, the optically active ketone compound (2) is induced, for example, according to the method described in JP-T-H07-505895 and the like, and thus it is possible to efficiently produce a 1-piperazino-1,2-dihydroindene derivative represented by the following formula (8): 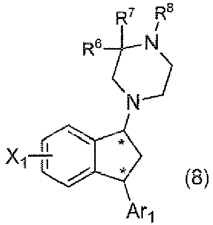 such as a trans-4-((1R,3S)-6-chloro-3-phenylindan-1-yl)-2,2-dimethylp iperazine salt from the enone compound (1).

In the formula (8), Ar₁ and X₁ represent the same as the above. * indicates an asymmetric carbon atom.

R⁶, R⁷ and R⁸ are independent of each other and represent a hydrogen atom, a C₁₋₁₂ alkyl group, an alkenyl group (for example, a C₂₋₁₂ alkenyl group), a C₆₋₁₄ aryl group, a C₇₋₁₅ aralkyl group, a cycloalkyl group (for example, a C₃₋₈ cycloalkyl group), or a cycloalkylalkyl group. These may have one or two or more substituents (nitro groups; hydroxy groups; halogen atoms such as a fluoro group, a chloro group, a bromo group, and an iodo group; haloalkyl groups; alkoxy groups; alkyl groups, aryl groups, etc.). Examples of R⁶, R⁷ and R⁸ that may have a substituent include a hydrogen atom, a phenyl group, a 4-chlorophenyl group, a 3-chlorophenyl group, a 2-chlorophenyl group, a 4-fluorophenyl group, a 3-fluorophenyl group, a 2-fluorophenyl group, a 4-bromophenyl group, a 3-bromophenyl group, a 2-bromophenyl group, a 4-trifluoromethylphenyl group, a 3-trifluoromethylphenyl group, a 2-trifluoromethylphenyl group, a 1-naphthyl group, a 2-naphthyl group, a 4-ethylphenyl group, a 3-ethylphenyl group, a 2-ethylphenyl group, a 4-methoxyphenyl group, a 3-methoxyphenyl group, a 2-methoxyphenyl group, a 4-phenylphenyl group, a methyl group, an ethyl group, a n-propyl group, an isopropyl group, a n-butyl group, an isobutyl group, a tert-butyl group, a n-octyl group, a hydroxymethyl group, a chloromethyl group, a trifluoromethyl group, a benzyl group, a methylcyclopropyl group, a 4-bromobutyl group, a 3-bromobutyl group, a 2-bromobutyl group, a 5-bromopentyl group, a 4-bromopentyl group, a 3-bromopentyl group, a 2-bromopentyl group, a 2-phenylethyl group, a 1-phenylethyl group, a 3-phenylbutyl group, a 2-phenylbutyl group, a 1-phenylbutyl group, a p-hydroxyphenyl group, an o, m, p-nitrophenyl group, an o, m, p-tolyl group, a p-chlorobenzyl group, a 2,4,6-trimethylphenyl group, a 2,4,6-triisopropylphenyl group, a 2,4,6-trimethoxyphenyl group, and a 2,4,6-trichlorophenyl group.

R⁶ and R⁷, and R⁷ and R⁸ may be respectively linked with each other to form a ring. Specifically, R⁶ and R⁷ may be linked with each other and connected to the piperazine ring in spiro. R⁷ and R⁸ may be linked with each other to form a piperazine ring.

R⁶ and R⁷ are each preferably a methyl group. In addition, R⁸ is preferably a hydrogen atom or a methyl group, and particularly preferably a hydrogen atom.

### EXAMPLES

The present invention will be described below in more detail with reference to examples, but the present invention is not limited thereto. The following Examples 3 to 17 and 23 to 33 further illustrate the claimed invention.

### (Example 1) 2-Bromo-6-chloro-3-phenylindanone

A solution (2 ml) of 2.0 g of 6-chloro-3-phenylindanone in dichloromethane was cooled to 0°C, and 1.3 g of bromine was added. The reaction mixture was heated to room temperature, and further stirred at room temperature for 1 hour. After the reaction, dichloromethane and water were added to extract the product, and the resulting organic layer was dried over magnesium sulfate. Thereafter, magnesium sulfate was filtered, and the resulting filtrate was concentrated under reduced pressure to obtain 2.6 g of the titled compound (yield 96%). ¹H NMR (CDCl₃): δ 7.85 (d, 1H, J=2.0Hz), 7.61 (dd, 1H, J=2.2, 8.3Hz), 7.49-7.20 (m, 4H), 7.20 (d, 1H, J=8.3Hz), 7.25-7.07 (m, 1H), 4.66 (d, 1H, J=4.2Hz), 4.51 (d, 1H, J=4.2Hz).

### (Example 2) 6-Chloro-3-phenylindenone

### (Method 1)

To a solution (16 ml) of 2.6 g of 2-bromo-6-chloro-3-phenylindanone in acetone was added 1.6 g of triethylamine, and the mixture was stirred at room temperature for 21.5 hours. After the reaction, ethyl acetate and water were added to extract the product, and the resulting organic layer was dried over magnesium sulfate. Thereafter, magnesium sulfate was filtered, and the resulting filtrate was concentrated under reduced pressure to obtain 995 mg of the titled compound (yield 52%).

### (Method 2)

To a solution (4.5 ml) of 256 mg of 2-bromo-6-chloro-3-phenylindanone in DMF was added 223 mg of potassium carbonate, and the mixture was stirred at room temperature for 22 hours. After the reaction, toluene and water were added to extract the product, and further, the resulting organic layer was washed with water. Thereafter, the organic layer was dried over magnesium sulfate. Thereafter, magnesium sulfate was filtered, and the resulting filtrate was concentrated under reduced pressure to obtain 175 mg of the titled compound as it is (yield 90%).
¹H NMR (CDCl₃) : δ 7.70-7.60 (m, 2H), 7.56-7.45 (m, 4H), 7.40-7.33 (m, 1H), 7.31 (d, 1H, J=8.0Hz), 6.03 (s, 1H).

### (Example 3) (S,S)-6-Chloro-3-phenylindanol

To 101 mg of 6-chloro-3-phenylindenone were added 5.6 mg of a [RuCl((S,S)-tosyldiphenylethylenediamine)(p-cymene)]complex catalyst, 336 mg of triethylamine and 134 mg of formic acid, and the mixture was stirred at room temperature for 22 hours. After the reaction, ethyl acetate and water were added to extract the product. Thereafter, the organic layer was dried over magnesium sulfate. Thereafter, magnesium sulfate was filtered, and the resulting filtrate was concentrated under reduced pressure to obtain a crude product. The resulting crude product was purified using a silica gel column to obtain 82.5 mg of 6-chloro-3-phenylindanol (yield 83%). The ratio of (S,S)-enantiomer/(S,R)-enantiomer by the following NMR was 79/21. Also, the optical purity of the titled compound by the following HPLC was 83% ee.
¹H NMR (CDCl₃) : δ 7.67-7.25 (m, 1H), 7.37-7.14 (m, 5H), 6.74-6.84 (m, 1H), 5.26 (dd, 1H, J=7.3, 7,3Hz), 4.14 (t, 1H, J=8.5Hz), 3.04 (dt, 1H, J=7.3, 12.7Hz), 2.02-1.90 (m, 1H). Determination method of the ratio of (S,S)-enantiomer/(S,R)-enantiomer: NMR method

In ¹H NMR, the ratio is calculated from the ratio of the integrated intensity of a peak at 5.26 ppm (dd) derived from the (S,S)-enantiomer to a peak at 5.36 ppm derived from the (S,R)-enantiomer.

### Determination method of the optical purity: HPLC

HPLC analysis is performed in the following conditions, and the optical purity is calculated from the peak areas of the (S,S)-enantiomer and (S,R)-enantiomer.
Column: Chiral Pack OD-H (registered trademark; manufactured by Daicel Corporation)
Eluent: n-hexane/isopropanol = 90/10 (volume ratio)
Flow rate: 1.0 mL/minute
Detection: 254 nm
Column temperature: 30°C
Detection time: (S,S)-enantiomer: 6.8 minutes. (R,R)-enantiomer: 19.7 minutes.

### (Reference Example 1) Preparation of transformant producing OYE3 derived from Saccharomyces cerevisiae

A transformant producing OYE3 derived from the Saccharomyces cerevisiae S288C (ATCC26108) strain was prepared in the same manner as in the method for preparing a transformant producing OYE2 derived from the Saccharomyces cerevisiae S288C (ATCC26108) strain described in Example 5 of International Publication WO 2006/129628. Using primer 1: 5'-ATCGAGCTCTTATCAGTTCTTGTTCCAACC-3' (SEQ ID NO:1 in the sequence listing) and primer 2: 5'-ACGCGTCGACTTATCAGTTCTTGTTCCAACCTAAA-3' (SEQ ID NO:2 in the sequence listing), a double-stranded DNA in which an SacI site was added to the initiation codon site of DNA encoding OYE3 derived from Saccharomyces cerevisiae and a new termination codon and an SalI site were added to just after the termination codon was amplified, and inserted into plasmid pUCNT (International Publication WO 94/03613). E. coli HB101 was transformed with this plasmid pTSYE3 to prepare transformant E. coli HB101 (pTSYE3) producing OYE3.

### (Reference Example 2) Preparation of transformant producing KYE derived from Kluyveromyces lactis

A transformant producing KYE derived from the Kluyveromyces lactis NBRC1267 strain was prepared in the same manner as in Reference Example 1. Using primer 3: 5'-AATATATACATATGTCGTTTATGAACTTTGAACCAAAGCC-3' (SEQ ID NO:3 in the sequence listing) and primer 4: 5'-ATATGAGCTCTTACTATTTCTTGTAACCCTTGGCAACAGCTTCC-3' (SEQ ID NO:4 in the sequence listing), a double-stranded DNA in which an NdeI site was added to the initiation codon site of DNA encoding KYE derived from Kluyveromyces lactis and a new termination codon and an SacI site were added to just after the termination codon was amplified, and inserted into plasmid pUCNl8 (a plasmid constructed by replacing "T" at the 185th position with "A" of pUC18 (manufactured by Takara Bio Inc., GenBank Accession No. L09136) by a PCR method to destroy the NdeI site, and further replacing "GC" at the 471st to 472nd positions with "TG" to introduce a new NdeI site) . E. coli HB101 was transformed with this plasmid pNKYE to prepare transformant E. coli HB101 (pNKYE) producing KYE.

### (Reference Example 3) Preparation of transformant producing YqjM derived from Bacillus subtilis

A transformant producing YqjM derived from the Bacillus subtilis JCM10629 strain was prepared in the same manner as in Reference Example 2. Using primer 5: 5'-ATATATACATATGGCCAGAAAATTATTTACACCTATTAC-3' (SEQ ID NO: 5 in the sequence listing) and primer 6: 5'-ATATGAGCTCTTATTACCAGCCTCTTTCGTATTGAACAGGG-3' (SEQ ID NO:6 in the sequence listing), a double-stranded DNA in which an NdeI site was added to the initiation codon site of DNA encoding YqjM derived from Bacillus subtilis and a new termination codon and an SacI site were added to just after the termination codon was amplified, and inserted into plasmid pUCN18. E. coli HB101 was transformed with this plasmid pNYqjM to prepare transformant E. coli HB101 (pNYqjM) producing YqjM.

### (Reference Example 4) Preparation of transformant producing NemA derived from Escherichia coli

A transformant producing NemA derived from the Escherichia coli NBRC3301 strain was prepared in the same manner as in Reference Example 2. Using primer 7: 5'-ATAGAATTCTAAGGAGGTTAACAATGTCATCTGAAAAACTGTATT-3' (SEQ ID NO:7 in the sequence listing) and primer 8: 5'-ATATGGTACCTTATTACAACGTCGGGTAATCGGTATAGCC-3' (SEQ ID NO:8 in the sequence listing), a double-stranded DNA in which an EcoRI site was added to just before the initiation codon of DNA encoding NemA derived from Escherichia coli and a new termination codon and a KpnI site were added to just after the termination codon was amplified, and inserted into plasmid pUCN18. E. coli HB101 was transformed with this plasmid pNNemA to prepare transformant E. coli HB101 (pNNemA) producing NemA.

### (Reference Example 5) Preparation of transformant producing XenA derived from Pseudomonas putida

A transformant producing XenA derived from the Pseudomonas putida NBRC100650 strain was prepared in the same manner as in Reference Example 2. Using primer 9: 5'-ATATATACATATGTCCGCACTGTTCGAACCCTACACCCTC-3' (SEQ ID NO:9 in the sequence listing) and primer 10: 5'-ATATGAGCTCTTATCAGCGATAACGCTCGAGCCAGTGTGCATAAG-3' (SEQ ID NO:10 in the sequence listing), a double-stranded DNA in which an NdeI site was added to the initiation codon site of DNA encoding XenA derived from Pseudomonas putida and a new termination codon and an SacI site were added to just after the termination codon was amplified, and inserted into plasmid pUCN18. E. coli HB101 was transformed with this plasmid pNXenA to prepare transformant E. coli HB101 (pNXenA) producing XenA.

### (Reference Example 6) Preparation of transformant producing XenE derived from Pseudomonas putida

A transformant producing XenE derived from the Pseudomonas putida NBRC100650 strain was prepared in the same manner as in Reference Example 2. Using primer 11: 5'-ATATATACATATGAGCCTGCTGCTCGAGCCTTACACCC-3' (SEQ ID NO:11 in the sequence listing) and primer 12: 5'-ATATGAGCTCTTATTAATCCCGCAAGTCCGACTCATGTATCGG-3' (SEQ ID NO:12 in the sequence listing), a double-stranded DNA in which an NdeI site was added to the initiation codon site of DNA encoding ZenE derived from Pseudomonas putida and a new termination codon and an SacI site were added to just after the termination codon was amplified, and inserted into plasmid pUCN18. E. coli HB101 was transformed with this plasmid pNXenE to prepare transformant E. coli HB101 (pNXenE) producing XenE.

### (Reference Example 7) Preparation of transformant producing YersER derived from Yersinia bercovieri

A transformant producing YersER derived from the Yersinia bercovieri NBRC105717 strain was prepared in the same manner as in Reference Example 2. Using primer 13: 5'-ATATATACATATGAAGACTGCTAAACTGTTCTCTCC-3' (SEQ ID NO:13 in the sequence listing) and primer 14: 5'-ATATGAGCTCTTATTACAGCGTTGGGTAATCAGTGTAGCC-3' (SEQ ID NO:14 in the sequence listing), a double-stranded DNA in which an NdeI site was added to the initiation codon site of DNA encoding YersER derived from Yersinia bercovieri and a new termination codon and an SacI site were added to just after the termination codon was amplified, and inserted into plasmid pUCN18. E. coli HB101 was transformed with this plasmid pNYersER to prepare transformant E. coli HB101 (pNYersER) producing YersER.

### (Reference Example 8) Preparation of transformant producing NCR-R derived from Zymomonas mobilis

A transformant producing NCR-R derived from the Zymomonas mobilis NBRC3301 strain was prepared in the same manner as in Reference Example 2. Using primer 15: 5'-ATAGAATTCTAAGGAGGTTAACAATGCCTAGCTTGTTTGATCCC-3' (SEQ ID NO:15 in the sequence listing) and primer 16: 5'-ATATGGTACCTTATCAATCCCCAAGCAAAGGATAATC-3' (SEQ ID NO:16 in the sequence listing), a double-stranded DNA in which an EcoRI site was added to just before the initiation codon of DNA encoding NCR-R derived from Zymomonas mobilis and a new termination codon and a KpnI site were added to just after the termination codon was amplified, and inserted into plasmid pUCN18. E. coli HB101 was transformed with this plasmid pNNCR-R to prepare transformant E. coli HB101 (pNNCR-R) producing NCR-R.

### (Example 4) Production of (S)-6-chloro-3-phenylindanone using OYE2 derived from Saccharomyces cerevisiae

In a large test tube was dispensed 5 mL of a liquid culture medium (pH = 7) containing 16 g of tripton, 10 g of an yeast extract, and 5 g of NaCl (per liter each), and the large test tube was sterilized by steam at 120°C for 20 minutes. To the liquid culture medium were aseptically inoculated a loopful of E. coli HB101 (pTSYE2) (see Example 7 of International Publication WO 2006/129628) producing OYE2 derived from Saccharomyces cerevisiae and a loopful of E. coli HB101 (pNTG1) (see Example 3 of International Publication WO 2006/033333) producing a glucose dehydrogenase derived from Bacillus megaterium, and the culture medium was cultured under shaking at 37°C for 24 hours.

After the culturing, the microbial cells were concentrated by centrifugation, and then disrupted by an ultrasonic homogenizer. Then, 0.1 ml of a disrupted liquid of the concentrated microbial cells of E. coli HB101 (pTSYE2), 0.1 ml of a disrupted liquid of the concentrated microbial cells of E. coli HB101 (pNTG1), 10 mg of 6-chloro-3-phenylindenone, each 1 mg of NAD and NADP, 20 mg of glucose, and 0.8 ml of a 0.1 M phosphate buffer solution (pH 6.5) were added, and the mixture was allowed to react at 30°C for 24 hours. In addition, as a control experiment, the disrupted liquid of the concentrated microbial cells of E. coli HB101 (pNTG1) was only added, and the mixture was allowed to react in the same manner as described above without adding the disrupted liquid of the concentrated microbial cells of E. coli HB101 (pTSYE2).

After the reaction, the reaction mixture was extracted with a double volume of ethyl acetate, and the organic layer was diluted, and analyzed by high performance liquid chromatography. The conversion rate and the optical purity of the produced (S)-6-chloro-3-phenylindanone were calculated.

In the reaction by adding the disrupted liquid of the concentrated microbial cells of E. coli HB101 (pTSYE2), the conversion rate of 6-chloro-3-phenylindenone into 6-chloro-3-phenylindanone was 88%, and the optical purity of the produced (S)-6-chloro-3-phenylindanone was 97.6% e.e. On the other hand, in the reaction by adding only the disrupted liquid of the concentrated microbial cells of E. coli HB101 (pNTG1), the conversion rate into 6-chloro-3-phenylindanone was 3%, and the conversion rate into 6-chloro-3-phenylindenol was 2%. The optical purities of the produced (R)-6-chloro-3-phenylindanone and (R)-6-chloro-3-phenylindenol were both 99.9% e.e. or more.

### <Analysis conditions of high performance liquid chromatography>

Column: CHIRALCEL OD-H manufactured by Daicel Corporation (250 mm × 4.6 mm)
Eluent: n-hexane/2-propanol = 95/5
Flow rate: 1.0 ml/minute
Detection: 254 nm
Column temperature: 30°C
Detection time: 6-Chloro-3-phenylindenone: 8.7 minutes, (S)-6-chloro-3-phenylindanone: 9.5 minutes, (R)-6-chloro-3-phenylindanone: 12.0 minutes, (R)-6-chloro-3-phenylindenol: 14.8 minutes, (S)-6-chloro-3-phenylindenol: 33.7 minutes

### (Example 5) Production of (S)-6-chloro-3-phenylindanone using OYE3 derived from Saccharomyces cerevisiae

The reaction and analysis were carried out in the same manner as in Example 4, using E. coli HB101 (pTSYE3) prepared in Reference Example 1 in place of E. coli HB101 (pTSYE2). As a result, the conversion rate was 85%, and the optical purity of the produced (S)-6-chloro-3-phenylindanone was 96.3% e.e.

### (Example 6) Production of (S)-6-chloro-3-phenylindanone using baker's yeast

In a large test tube were dispensed 0.5 g of Algist Bruggeman NV baker's yeast (trade name: Bruggeman Instant Yeast Blue), 1 g of glucose, and 10 ml of water, and the large test tube was shaken at 30°C for 1 hour. To 1 ml of this yeast suspension were added 10 mg of 6-chloro-3-phenylindenone and 20 mg of glucose, and the mixture was adjusted to a pH of 7 with sodium hydroxide and allowed to react at 30°C for 24 hours. After the reaction, the product was analyzed in the same manner as in Example 4 to calculate the conversion rate and the optical purity. As a result, the conversion rate was 54%, and the optical purity of the produced (S)-6-chloro-3-phenylindanone was 99.2% e.e.

### (Example 7) Production of (S)-6-chloro-3-phenylindanone using OYE2 derived from Saccharomyces cerevisiae

E. coli HB101 (pTSYE2) and E. coli HB101 (pNGLP2) (see Example 5 of International Publication WO 2009/041415) producing a glucose dehydrogenase derived from Lactobacillus pentosus were cultured in the same manner as in Example 4, and the reaction was carried out in the same manner as in Example 4. In addition, as a control experiment, the reaction of only the disrupted liquid of the concentrated microbial cells of E. coli HB101 (pNGLP2) was carried out without using E. coli HB101 (pTSYE2). When the analysis was carried out in the same manner as in Example 4, the conversion rate was 99% in the reaction by adding the disrupted liquid of the concentrated microbial cells of E. coli HB101 (pTSYE2), and the optical purity of the produced (S)-6-chloro-3-phenylindanone was 99.9% e.e. or more. On the other hand, in the reaction by adding only E. coli HB101 (pNGLP2), production of 6-chloro-3-phenylindanone and 6-chloro-3-phenylindenol was not found.

The use of the glucose dehydrogenase derived from Lactobacillus pentosus in place of the glucose dehydrogenase derived from Bacillus megaterium resulted in producing (S)-6-chloro-3-phenylindanone having optical purity higher than that in Example 4. In addition, the conversion rate was higher than that of the case of using bakery's yeast in Example 6.

### (Example 8) Production of (S)-6-chloro-3-phenylindanone using KYE derived from Kluyveromyces lactis

The reaction and analysis were carried out in the same manner as in Example 7, using E. coli HB101 (pNKYE) prepared in Reference Example 2 in place of E. coli HB101 (pTSYE2) . As a result, the conversion rate was 94%, and the optical purity of the produced (S)-6-chloro-3-phenylindanone was 99.9% e.e.

### (Example 9) Production of (S)-6-chloro-3-phenylindanone using YqjM derived from Bacillus subtilis

The reaction and analysis were carried out in the same manner as in Example 7, using E. coli HB101 (pNYqjM) prepared in Reference Example 3 in place of E. coli HB101 (pTSYE2). As a result, the conversion rate was 38%, and the optical purity of the produced (S)-6-chloro-3-phenylindanone was 98.9% e.e.

### (Example 10) Production of (S)-6-chloro-3-phenylindanone using NemA derived from Escherichia coli

The reaction and analysis were carried out in the same manner as in Example 7, using E. coli HB101 (pNNemA) prepared in Reference Example 4 in place of E. coli HB101 (pTSYE2). As a result, the conversion rate was 90%, and the optical purity of the produced (S)-6-chloro-3-phenylindanone was 99.9% e.e.

### (Example 11) Production of (S)-6-chloro-3-phenylindanone using XenA derived from Pseudomonas putida

The reaction and analysis were carried out in the same manner as in Example 7, using E. coli HB101 (pNXenA) prepared in Reference Example 5 in place of E. coli HB101 (pTSYE2) . As a result, the conversion rate was 4%, and the optical purity of the produced (S)-6-chloro-3-phenylindanone was 56.6% e.e.

### (Example 12) Production of (S)-6-chloro-3-phenylindanone using XenE derived from Pseudomonas putida

The reaction and analysis were carried out in the same manner as in Example 7, using E. coli HB101 (pNpNXenE) prepared in Reference Example 6 in place of E. coli HB101 (pTSYE2) . As a result, the conversion rate was 3%, and the optical purity of the produced (S)-6-chloro-3-phenylindanone was 12.9% e.e.

### (Example 13) Production of (S)-6-chloro-3-phenylindanone using YersER derived from Yersinia bercovieri

The reaction and analysis were carried out in the same manner as in Example 7, using E. coli HB101 (pNYersER) prepared in Reference Example 7 in place of E. coli HB101 (pTSYE2) . As a result, the conversion rate was 39%, and the optical purity of the produced (S)-6-chloro-3-phenylindanone was 98.9% e.e.

### (Example 14) Production of (S)-6-chloro-3-phenylindanone using NCR-R derived from Zymomonas mobilis

The reaction and analysis were carried out in the same manner as in Example 7, using E. coli HB101 (pNNCR-R) prepared in Reference Example 8 in place of E. coli HB101 (pTSYE2) . As a result, the conversion rate was 90%, and the optical purity of the produced (S)-6-chloro-3-phenylindanone was 99.9% e.e.

### (Example 15) Production of (S)-6-chloro-3-phenylindanone using OYE2 derived from Saccharomyces cerevisiae

The reaction was carried out in the same manner as in Example 7 using E. coli HB101 (pTSYE2) and E. coli HB101 (pNGLP) (see Example 5 of International Publication WO 2009/041415) producing a glucose dehydrogenase derived from Lactobacillus plantarum. When the analysis was carried out in the same manner as in Example 4, the conversion rate was 99%, and the optical purity of the produced (S) -6-chloro-3-phenylindanone was 99.9% e.e. or more.

### (Example 16) Production of (S)-6-chloro-3-phenylindanone using OYE2 derived from Saccharomyces cerevisiae

The reaction was carried out in the same manner as in Example 7 using E. coli HB101 (pTSYE2) and E. coli HB101 (pNGLP) (see Example 14 of International Publication WO 2009/041415) producing a glucose dehydrogenase derived from Pediococcus parvulus. When the analysis was carried out in the same manner as in Example 4, the conversion rate was 99%, and the optical purity of the produced (S) -6-chloro-3-phenylindanone was 99.9% e.e. or more.

### (Example 17) Production of (S)-6-chloro-3-phenylindanone using OYE2 derived from Saccharomyces cerevisiae

E. coli HB101 (pTSYE2) and E. coli HB101 (pNGLP2) producing a glucose dehydrogenase derived from Lactobacillus pentosus were cultured in the same manner as in Example 4, and after the culturing, the microbial cells were concentrated by centrifugation, and then disrupted by an ultrasonic homogenizer. To 4 ml of a disrupted liquid of the concentrated microbial cells of each transformant were added 2 g of 6-chloro-3-phenylindenone, 4 mg of NADP⁺, 4 g of glucose, and 32 ml of a 0.1 M phosphate buffer solution (pH 6.5), and the mixture was allowed to react at 30°C for 40 hours while stirring. During the reaction, the reaction mixture was maintained at a pH of 6.5 with 5 N NaOH. After 40 hours, when the analysis was carried out in the same manner as in Example 4, the conversion rate was 98%, and the optical purity of the produced (S)-6-chloro-3-phenylindanone was 99.9% e.e. or more.

Next, (S)-6-chloro-3-phenylindanone was extracted from the reaction mixture with ethyl acetate. The solvent was distilled off under reduced pressure to obtain 1.9 g of (S)-6-chloro-3-phenylindanone.

### (Example 18) Production of (S)-6-chloro-3-phenylindenol using carbonyl reductase derived from Candida magnoliae

E. coli HB101 (pNTCR) (described in Example 5 of JP Patent 4510351) producing a carbonyl reductase derived from Candida magnoliae was cultured in the same manner as in Example 4. After the culturing, the microbial cells were disrupted by an ultrasonic homogenizer, then 0.9 ml of each disrupted liquid of the microbial cells, 10 mg of 6-chloro-3-phenylindenone, each 1 mg of NAD and NADP, 20 mg of glucose, 5 U of a glucose dehydrogenase (trade name: GLUCDH "Amano" II, manufactured by Amano Enzyme Inc.) and 0.1 ml of a 1 M phosphate buffer solution (pH 6.5) were added, and the mixture was allowed to react at 30°C for 24 hours.

After the reaction, the analysis was carried out in the same manner as in Example 4, and the optical purity of the produced (S)-6-chloro-3-phenylindenol was calculated to be 52.4% e.e.

### (Example 19) Production of (S)-6-chloro-3-phenylindenol using carbonyl reductase derived from Ogataea minuta var. minuta

The reaction of 6-chloro-3-phenylindenone was carried out in the same manner as in Example 18, using E. coli HB101 (pNTOM5) (described in Example 6 of International Publication WO 2006/013801) producing a carbonyl reductase derived from Ogataea minuta var. minuta. The analysis was carried out in the same manner as in Example 4, and the optical purity of the produced (S)-6-chloro-3-phenylindenol was 84.4% e.e.

### (Example 20) Production of (S)-6-chloro-3-phenylindenol using carbonyl reductase derived from Brevundimonas diminuta

The reaction of 6-chloro-3-phenylindenone was carried out in the same manner as in Example 18, using E. coli HB101 (pNBD) (described in Example 8 of International Publication WO 2007/114217) producing a carbonyl reductase derived from Brevundimonas diminuta. The analysis was carried out in the same manner as in Example 4, and the optical purity of the produced (S)-6-chloro-3-phenylindenol was 96.1% e.e.

### (Example 21) Production of (R)-6-chloro-3-phenylindenol using carbonyl reductase derived from Ogataea minuta var. minuta

The reaction of 6-chloro-3-phenylindenone was carried out in the same manner as in Example 18, using E. coli HB101 (pNTOM3) (described in Example 8 of International Publication WO 2006/013801) producing a carbonyl reductase derived from Ogataea minuta var. minuta. The analysis was carried out in the same manner as in Example 4, and the optical purity of the produced (R)-6-chloro-3-phenylindenol was 66.3% e.e.

### (Example 22) Production of (R)-6-chloro-3-phenylindenol using glucose dehydrogenase derived from Bacillus megaterium

The reaction of 6-chloro-3-phenylindenone was carried out in the same manner as in Example 18, using E. coli HB101 (pNTG1) producing a glucose dehydrogenase derived from Bacillus megaterium used in Example 4. The analysis was carried out in the same manner as in Example 4, and the optical purity of the produced (R)-6-chloro-3-phenylindenol was 92.1% e.e.

### (Example 23) Production of (S,S)-6-chloro-3-phenylindanol using carbonyl reductase derived from Candida magnoliae

E. coli HB101 (pNTCR) producing a carbonyl reductase derived from Candida magnoliae (described in Example 5 of JP Patent 4510351) was cultured in the same manner as in Example 4. After the culturing, the microbial cells were disrupted by an ultrasonic homogenizer, then 0.9 ml of the disrupted liquid of the microbial cells, 10 mg of (S)-6-chloro-3-phenylindanone (99.9% e.e. or more), each 1 mg of NAD and NADP, 20 mg of glucose, 5 U of a glucose dehydrogenase (trade name: GLUCDH "Amano" II, manufactured by Amano Enzyme Inc .) and 0.1 ml of a 1 M phosphate buffer solution (pH 6.5) were added, and the mixture was allowed to react at 30°C for 24 hours. As a control experiment, the reaction without adding the disrupted liquid of the microbial cells was also carried out.

After the reaction, the reaction mixture was extracted with a double volume of ethyl acetate, and the organic layer was diluted and analyzed by high performance liquid chromatography to calculate the ratio of (S, S) -enantiomer/ (S, R) -enantiomer of the produced 6-chloro-3-phenylindanol.

As a result, in the reaction by adding E. coli HB101 (pNTCR), the conversion rate into 6-chloro-3-phenylindanol was 28%, and the ratio of (S,S)-enantiomer/(S,R)-enantiomer was 100/0. On the other hand, in the control experiment without adding E. coli HB101 (pNTCR), production of 6-chloro-3-phenylindanol could not be found.

### <Analysis conditions of high performance liquid chromatography>

Column: Finepak SIL C18T-5 manufactured by JASCO Corporation (250 mm × 4.6 mm)
Eluent: 10 mM potassium phosphate buffer/acetonitrile = 3/7
Flow rate: 1.0 ml/minute
Detection: 254 nm
Detection time: (S)-6-Chloro-3-phenylindanone: 17.1 minutes, (S,R)-6-chloro-3-phenylindanol: 13.1 minutes, (S,S)-6-chloro-3-phenylindanol 14.4 minutes

### (Example 24) Production of (S,S) -6-chloro-3-phenylindanol using carbonyl reductase derived from Candida maris

The reaction of (S)-6-chloro-3-phenylindanone was carried out in the same manner as in Example 23, using E. coli HB101 (pNTFP) (described in Example 24 of International Publication WO 2001/05996) producing a carbonyl reductase derived from Candida maris. The analysis was carried out in the same manner as in Example 23, and the ratio of (S,S)-enantiomer/(S,R)-enantiomer of the produced 6-chloro-3-phenylindanol was 100/0.

### (Example 25) Production of (S,S)-6-chloro-3-phenylindanol using carbonyl reductase derived from Devosia riboflavina

The reaction of (S)-6-chloro-3-phenylindanone was carried out in the same manner as in Example 23, using E. coli HB101 (pNTDR) (described in Example 6 of JP Patent 4414337) producing a carbonyl reductase derived from Devosia riboflavina. The analysis was carried out in the same manner as in Example 23, and the ratio of (S,S)-enantiomer/(S,R)-enantiomer of the produced 6-chloro-3-phenylindanol was 100/0.

### (Example 26) Production of (S,S)-6-chloro-3-phenylindanol using carbonyl reductase derived from Candida magnoliae

The reaction of (S)-6-chloro-3-phenylindanone was carried out in the same manner as in Example 23, using E. coli HB101 (pNTCM) (described in Example 5 of International Publication WO 2006/046455) producing a carbonyl reductase derived from Candida magnoliae. The analysis was carried out in the same manner as in Example 23, and the ratio of (S,S)-enantiomer/(S,R)-enantiomer of the produced 6-chloro-3-phenylindanol was 100/0.

### (Example 27) Production of (S,S)-6-chloro-3-phenylindanol using carbonyl reductase derived from Candida maltosa

The reaction of (S)-6-chloro-3-phenylindanone was carried out in the same manner as in Example 23, using E. coli HB101 (pNCM) (described in Example 8 of International Publication WO 2008/066018) producing a carbonyl reductase derived from Candida maltosa. The analysis was carried out in the same manner as in Example 23, and the ratio of (S,S)-enantiomer/(S,R)-enantiomer of the produced 6-chloro-3-phenylindanol was 100/0.

### (Example 28) Production of (S,S)-6-chloro-3-phenylindanol using carbonyl reductase derived from Ogataea minuta var. minuta

The reaction of (S)-6-chloro-3-phenylindanone was carried out in the same manner as in Example 23, using E. coli HB101 (pNTOM5) (described in Example 6 of International Publication WO 2006/013801) producing a carbonyl reductase derived from Ogataea minuta var. minuta. The analysis was carried out in the same manner as in Example 23, and the ratio of (S,S)-enantiomer/(S,R)-enantiomer of the produced 6-chloro-3-phenylindanol was 100/0.

### (Example 29) Production of (S,S)-6-chloro-3-phenylindanol using carbonyl reductase derived from Saccharomyces cerevisiae

The reaction of (S)-6-chloro-3-phenylindanone was carried out in the same manner as in Example 23, using E. coli HB101 (pNSC1) producing a carbonyl reductase derived from Saccharomyces cerevisiae (described in Reference Example 1 of JP-A-2010-130912). The analysis was carried out in the same manner as in Example 23, and the ratio of (S,S)-enantiomer/(S,R)-enantiomer of the produced 6-chloro-3-phenylindanol was 100/0.

### (Example 30) Production of (S,S)-6-chloro-3-phenylindanol using carbonyl reductase derived from Brevundimonas diminuta

The reaction of (S)-6-chloro-3-phenylindanone was carried out in the same manner as in Example 23, using E. coli HB101 (pNBD) (described in Example 8 of International Publication WO 2007/114217) producing a carbonyl reductase derived from Brevundimonas diminuta. The analysis was carried out in the same manner as in Example 23, and the ratio of (S,S)-enantiomer/(S,R)-enantiomer of the produced 6-chloro-3-phenylindanol was 100/0.

### (Example 31) Production of (S,S)-6-chloro-3-phenylindanol using carbonyl reductase derived from Paenibacillus alvei

The reaction of (S)-6-chloro-3-phenylindanone was carried out in the same manner as in Example 23, using E. coli HB101 (pNTBA) (described in Example 4 of International Publication WO 2007/099764) producing a carbonyl reductase derived from Paenibacillus alvei. The analysis was carried out in the same manner as in Example 23, and the ratio of (S,S)-enantiomer/(S,R)-enantiomer of the produced 6-chloro-3-phenylindanol was 100/0.

### (Example 32) Production of (S,S)-6-chloro-3-phenylindanol using carbonyl reductase derived from Pseudomonas stutzeri

The reaction of (S)-6-chloro-3-phenylindanone was carried out in the same manner as in Example 23, using E. coli HB101 (pNPS) (described in Example 5 of International Publication WO 2007/099994) producing a carbonyl reductase derived from Pseudomonas stutzeri . The analysis was carried out in the same manner as in Example 23, and the ratio of (S,S)-enantiomer/(S,R)-enantiomer of the produced 6-chloro-3-phenylindanol was 100/0.

### (Example 33) (S,S)-6-Chloro-3-phenylindanone

To 100 mg of a solution of 6-chloro-3-phenylindenone in methanol were added 5.6 mg of a [RuCl((S,S)-tosyldiphenylethylenediamine) (p-cymene)]complex catalyst, 168 mg of triethylamine and 67 mg of formic acid, and the mixture was stirred at room temperature for 16 hours . After the reaction, ethyl acetate and water were added to extract the product. Thereafter, the organic layer was dried over magnesium sulfate. Thereafter, magnesium sulfate was filtered, and the resulting filtrate was concentrated under reduced pressure to obtain 6-chloro-3-phenylindanone in a yield of 30%. Also, the optical purity of the titled compound was 47% ee. ¹H NMR (CDCl₃): δ 7.76 (d, 1H, J=2.2, 7.8Hz), 7.51 (dd, 1H, J=2.0, 8.3Hz), 7.42-7.12 (m, 4H), 7.20-7.04 (m, 2H), 4.54 (dd, 1H, J=3.9, 8.0Hz), 3.26 (dd, 1H, J=8.0, 19.3Hz), 2.72 (dd, 1H, J=3.9, 19.3Hz).

### INDUSTRIAL APPLICABILITY

The present invention can be used for producing an optically active compound having an indene or indane skeleton (such as optically active indanones, optically active indenols, and optically active indanols). Such an optically active compound is useful as a pharmaceutical intermediate, and can be used, for example, for producing a trans-4-((1R,3S)-6-chloro-3-phenylindan-1-yl)-2,2-dimethylp iperazine salt that is used in an agent for schizophrenia.

## Claims

1. A method for producing an optically active ketone, comprising the step of:
stereoselectively reducing a carbon-carbon double bond of an enone site of an enone compound represented by the following formula (1) by means of an enzyme source or an asymmetric metal catalyst: 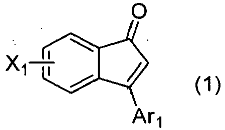 wherein Ar₁ represents a furyl group, a thienyl group or a C₆₋₂₀ aryl group, wherein these groups are optionally substituted by one, two or more substituents; and X₁ represents a halogen atom,
to produce an optically active ketone represented by the following formula (2): 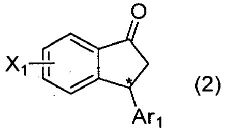 wherein Ar₁ and X₁ represent the same as the above, and * indicates an asymmetric carbon atom.

2. The method according to claim 1, wherein an enzyme source having an ability to stereoselectively reduce a carbon-carbon double bond of an enone site acts on.

3. The method according to claim 2, wherein the enzyme source is an Old Yellow Enzyme or an enone reductase.

4. The method according to claims 2 or 3, wherein the enzyme source is in the form of any one of microbial cells, a culture broth of the microorganism, a processed material thereof and an enzyme obtained from the microorganism,
and the microorganism is selected from the group consisting of the microorganism belonging to genus Saccharomyces, Kluyveromyces, Bacillus, Escherichia, Pseudomonas, Yersinia and Zymomonas.

5. The method according to claim 4, wherein the enzyme source is derived from the microorganism selected from the group consisting of Saccharomyces cerevisiae, Kluyveromyces lactis, Bacillus subtilis, Escherichia coli, Pseudomonas putida, Yersinia bercovieri and Zymomonas mobilis.

6. The method according to claim 1, wherein a carbon-carbon double bond of an enone site is stereoselectively reduced by an asymmetric metal catalyst.

7. The method according to claim 6, wherein the asymmetric metal catalyst comprises Ru, Ni, Rh, Pt, Pd, Ir or Cu.

8. The method according to claim 7, wherein the asymmetric metal catalyst is a Ru-, Rh-, Ir-, or Cu-based catalyst.

9. The method according to any one of claims 1-8,
further comprising stereoselectively reducing a carbonyl group of the optically active ketone represented by the formula (2),
to produce an optically active alcohol represented by the following formula (5): 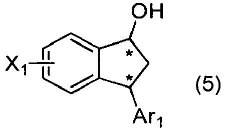 wherein Ar₁ and X₁ represent the same as the above, and * indicates an asymmetric carbon atom.

10. The method according to claim 9, wherein the carbonyl group is diastereoselectively reduced with use of a hydride reducing agent.

11. The method according to claim 9, wherein an enzyme source having an ability to stereoselectively reduce the carbonyl group acts on.

12. The method according to claim 11, wherein the enzyme source is in the form of any one of microbial cells, a culture broth of the microorganism, a processed material thereof and an enzyme obtained from the microorganism,
and the microorganism is selected from the group consisting of the microorganism belonging to genus Candida, Ogataea, Saccharomyces, Brevundimonas, Devosia, Paenibacillus and Pseudomonas.

13. The method according to claim 12, wherein the enzyme source is derived from a microorganism selected from the group consisting of Candida magnoliae, Candida maltosa, Candida maris, Ogataea minuta var.minuta, Saccharomyces cerevisiae, Brevundimonas diminuta, Devosia riboflavina, Paenibacillus alvei and Pseudomonas stutzeri.

14. The method according to any one of claims 2-5,
further comprising regenerating a coenzyme by an oxidoreductase,
wherein the oxidoreductase is an enzyme source not having an ability to reduce the carbon-carbon double bond of the enone site and the carbonyl group of the enone compound.

15. The method according to claim 14, wherein the oxidoreductase has activity of specifically acting on NADP⁺ to generate NADPH.

16. The method according to claim 14, wherein the oxidoreductase is derived from a lactic acid bacteria.

17. The method according to any one of claims 1-16, wherein the enone compound represented by the formula (1) is produced in the manner comprising steps of:
acting a halogenating agent on a ketone compound represented by the following formula (6): 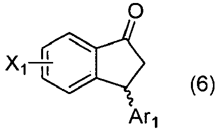 wherein Ar₁ and X₁ represent the same as the above, to produce a compound represented by the following formula (7): 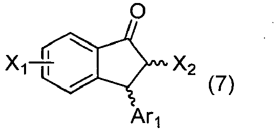 wherein Ar₁ and X₁ represent the same as the above; and X₂ represents a halogen atom, and
reacting the compound (7) with a base.

18. The method according to claim 17, wherein the halogenating agent is bromine.

19. The method according to any one of claims 17 or 18, wherein the base is a kind or more selected from the group consisting of triethylamine, diisopropylethylamine, pyridine, lithium carbonate, sodium carbonate, potassium carbonate, lithium hydroxide, sodium hydroxide, and potassium hydroxide.

## Patentansprüche

1. Ein Verfahren zur Herstellung eines optisch aktiven Ketons, umfassend den Schritt:
stereoselektives Reduzieren einer Kohlenstoff-Kohlenstoff-Doppelbindung einer Enonstelle einer Enonverbindung, dargestellt durch die folgende Formel (1), mittels einer Enzymquelle oder eines asymmetrischen Metallkatalysators: 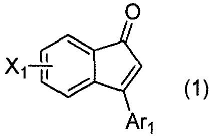 wobei Ar₁ eine Furylgruppe, eine Thienylgruppe oder eine C₆-C₂₀-Arylgruppe darstellt, wobei diese Gruppen gegebenenfalls mit einem, zwei oder mehreren Substituenten substituiert sind; und X₁ ein Halogenatom darstellt,
um ein optisch aktives Keton, dargestellt durch die folgende Formel (2), herzustellen: 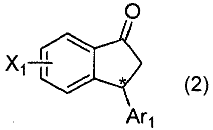 wobei Ar₁ und X₁ dasselbe wie oben darstellen und * ein asymmetrisches Kohlenstoffatom anzeigt.

2. Das Verfahren gemäß Anspruch 1, wobei eine Enzymquelle, welche eine Kohlenstoff-Kohlenstoff-Doppelbindung einer Enonstelle selektiv reduzieren kann, einwirkt.

3. Das Verfahren gemäß Anspruch 2, wobei die Enzymquelle ein "Old Yellow Enzyme" oder eine Enonreduktion ist.

4. Das Verfahren gemäß den Ansprüchen 2 oder 3, wobei die Enzymquelle in der Form von einer aus mikrobiellen Zellen, einer Kulturlösung des Mikroorganismus, einem verarbeiteten Material davon und einem aus dem Mikroorganismus erhaltenem Enzym vorliegt,
und der Mikroorganismus ausgewählt ist aus der Gruppe, bestehend aus dem Mikroorganismus, der zu den Stämmen Saccharomyces, Kluyveromyces, Bacillus, Escherichia, Pseudomonas, Yersinia und Zymomonas gehört.

5. Das Verfahren gemäß Anspruch 4, wobei die Enzymquelle aus dem Mikroorganismus, ausgewählt aus der Gruppe, bestehend aus Saccharomyces cerevisiae, Kluyveromyces lactis, Bacillus subtilis, Escherichia coli, Pseudomonas putida, Yersinia bercovieri und Zymomonas mobilis, abgeleitet ist.

6. Das Verfahren gemäß Anspruch 1, wobei eine Kohlenstoff-Kohlenstoff-Doppelbindung einer Enonstelle durch einen asymmetrischen Metallkatalysator stereoselektiv reduziert wird.

7. Das Verfahren gemäß Anspruch 6, wobei der asymmetrische Metallkatalysator Ru, Ni, Rh, Pt, Pd, Ir oder Cu umfasst.

8. Das Verfahren gemäß Anspruch 7, wobei der asymmetrische Metallkatalysator ein Katalysator auf der Basis von Ru, Rh, Ir oder Cu ist.

9. Das Verfahren gemäß einem der Ansprüche 1-8,
ferner umfassend stereoselektives Reduzieren einer Carbonylgruppe des optisch aktiven Ketons, dargestellt durch die Formel (2),
um einen optisch aktiven Alkohol, dargestellt durch die folgende Formel (5), herzustellen: 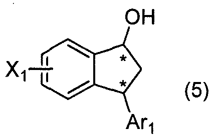 wobei Ar₁ und X₁ dasselbe wie oben darstellen und * ein asymmetrisches Kohlenstoffatom anzeigt.

10. Das Verfahren gemäß Anspruch 9, wobei die Carbonylgruppe unter Verwendung eines Hydrid reduzierenden Mittels diastereoselektiv reduziert wird.

11. Das Verfahren gemäß Anspruch 9, wobei eine Enzymquelle, welche die Carbonylgruppe selektiv reduzieren kann, einwirkt.

12. Das Verfahren gemäß Anspruch 11, wobei die Enzymquelle in der Form von einer aus mikrobiellen Zellen, einer Kulturlösung des Mikroorganismus, einem verarbeiteten Material davon und einem aus dem Mikroorganismus erhaltenen Enzym vorliegt,
und der Mikroorganismus ausgewählt ist aus der Gruppe, bestehend aus dem Mikroorganismus, der zu den Stämmen Candida, Ogataea, Saccharomyces, Brevundimonas, Devosia, Paenibacillus und Pseudomonas gehört.

13. Das Verfahren gemäß Anspruch 12, wobei die Enzymquelle von einem Mikroorganismus, ausgewählt aus der Gruppe bestehend aus Candida magnoliae, Candida maltosa, Candida maris, Ogataea minuta var.minuta, Saccharomyces cerevisiae, Brevundimonas diminuta, Devosia riboflavina, Paenibacillus alvei und Pseudomonas stutzeri abgeleitet ist.

14. Das Verfahren gemäß einem der Ansprüche 2-5,
ferner umfassend Regenerieren eines Coenzyms durch eine Oxidoreduktase,
wobei die Oxidoreduktase eine Enzymquelle ist, welche die Kohlenstoff-Kohlenstoff-Doppelbindung der Enonstelle und die Carbonylgruppe der Enonverbindung nicht reduzieren kann.

15. Das Verfahren gemäß Anspruch 14, wobei die Oxidoreduktase eine Aktivität hat, speziell auf NADP⁺ einzuwirken, um NADPH zu bilden.

16. Das Verfahren gemäß Anspruch 14, wobei die Oxidoreduktase aus einem Milchsäurebakterium abgeleitet ist.

17. Das Verfahren gemäß einem der Ansprüche 1-16, wobei die Enonverbindung, dargestellt durch die Formel (1), auf die Weise hergestellt ist, die die Schritte umfasst:
Einwirken eines halogenierenden Mittels auf eine Ketonverbindung, dargestellt durch die folgende Formel (6): 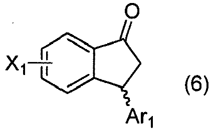 wobei Ar₁ und X₁ dasselbe wie oben darstellen, um eine Verbindung herzustellen, dargestellt durch die folgende Formel (7): 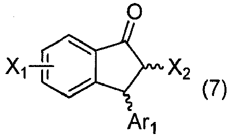 wobei Ar₁ und X₁ dasselbe wie oben darstellen; und X₂ ein Halogenatom darstellt, und
Umsetzen der Verbindung (7) mit einer Base.

18. Das Verfahren gemäß Anspruch 17, wobei das halogenierende Mittel Brom ist.

19. Das Verfahren gemäß einem der Ansprüche 17 oder 18, wobei die Base eine oder mehrere Arten, ausgewählt aus der Gruppe bestehend aus Triethylamin, Diisopropylethylamin, Pyridin, Lithiumcarbonat, Natriumcarbonat, Kaliumcarbonat, Lithiumhydroxid, Natriumhydroxid und Kaliumhydroxid, ist.

## Revendications

1. Méthode pour la production d'une cétone optiquement active, comprenant l'étape de :
réduction stéréosélective d'une double liaison carbone-carbone d'un site énone d'un composé énone représenté par la formule (1) suivante au moyen d'une source d'enzyme ou d'un catalyseur métallique asymétrique : 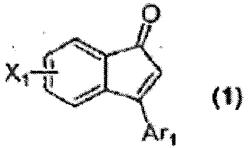 dans laquelle Ar₁ représente un groupe furyle, un groupe thiényle ou un groupe aryle en C₆ à C₂₀, dans laquelle ces groupes sont éventuellement substitués par un ou deux substituants ou plus ; et X₁ représente un atome d'halogène,
pour produire une cétone optiquement active représentée par la formule (2) suivante : 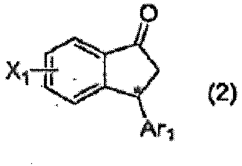 dans laquelle Ar₁ et X₁ représentent les mêmes groupes que ci-dessus, et * indique un atome de carbone asymétrique.

2. Méthode selon la revendication 1, dans laquelle une source d'enzyme ayant la capacité de réduire stéréosélectivement une double liaison carbone-carbone d'un site énone agit dessus.

3. Méthode selon la revendication 2, dans laquelle la source d'enzyme est une "Old Yellow Enzyme" ou une énone réductase.

4. Méthode selon les revendications 2 ou 3, dans laquelle la source d'enzyme est sous la forme de l'un(e) quelconque de cellules microbiennes, d'un bouillon de culture du micro-organisme, d'un matériau traité de celui-ci et d'une enzyme obtenue à partir du micro-organisme,
et le micro-organisme est choisi dans le groupe constitué du micro-organisme appartenant au genre Saccharomyces, Kluyveromyces, Bacillus, Escherichia, Pseudomonas, Yersinia et Zymomonas.

5. Méthode selon la revendication 4, dans laquelle la source d'enzyme est issue du micro-organisme choisi dans le groupe constitué de Saccharomyces cerevisiae, Kluyveromyces lactis, Bacillus subtilis, Escherichia coli, Pseudomonas putida, Yersinia bercovieri et Zymomonas mobilis.

6. Méthode selon la revendication 1, dans laquelle une double liaison carbone-carbone d'un site énone est réduite stéréosélectivement par un catalyseur métallique asymétrique.

7. Méthode selon la revendication 6, dans laquelle le catalyseur métallique asymétrique comprend Ru, Ni, Rh, Pt, Pd, Ir ou Cu.

8. Méthode selon la revendication 7, dans laquelle le catalyseur métallique asymétrique est un catalyseur à base de Ru, Rh, Ir, ou Cu.

9. Méthode selon l'une quelconque des revendications 1 à 8,
comprenant en outre la réduction stéréosélective d'un groupe carbonyle de la cétone optiquement active représentée par la formule (2),
pour produire un alcool optiquement actif représenté par la formule (5) suivante : 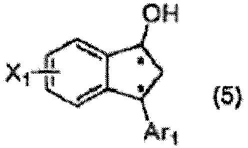 dans laquelle Ar₁ et X₁ représentent les mêmes groupes que ci-dessus, et * indique un atome de carbone asymétrique.

10. Méthode selon la revendication 9, dans laquelle le groupe carbonyle est réduit diastéréosélectivement au moyen d'un agent réducteur de type hydrure.

11. Méthode selon la revendication 9, dans laquelle une source d'enzyme ayant la capacité de réduire stéréosélectivement le groupe carbonyle agit dessus.

12. Méthode selon la revendication 11, dans laquelle la source d'enzyme est sous la forme de l'un(e) quelconque de cellules microbiennes, d'un bouillon de culture du micro-organisme, d'un matériau traité de celui-ci et d'une enzyme obtenue à partir du micro-organisme,
et le micro-organisme est choisi dans le groupe constitué du micro-organisme appartenant au genre Candida, Ogataea, Saccharomyces, Brevundimonas, Devosia, Paenibacillus et Pseudomonas.

13. Méthode selon la revendication 12, dans laquelle la source d'enzyme est issue d'un micro-organisme choisi dans le groupe constitué de Candida magnoliae, Candida maltosa, Candida maris, Ogataea minuta var. minuta, Saccharomyces cerevisiae, Brevundimonas diminuta, Devosia riboflavina, Paenibacillus alvei et Pseudomonas stutzeri.

14. Méthode selon l'une quelconque des revendications 2 à 5,
comprenant en outre la régénération d'un coenzyme par une oxydoréductase,
dans laquelle l'oxydoréductase est une source d'enzyme n'ayant pas la capacité de réduire la double liaison carbone-carbone du site énone et le groupe carbonyle du composé d'énone.

15. Méthode selon la revendication 14, dans laquelle l'oxydoréductase est dotée d'une activité d'action spécifique sur le NADP⁺ pour générer du NADPH.

16. Méthode selon la revendication 14, dans laquelle l'oxydoréductase est issue d'une bactérie lactique.

17. Méthode selon l'une quelconque des revendications 1 à 16, dans laquelle la production du composé énone représenté par la formule (1) est réalisée de manière qu'elle comprend les étapes d'(de) :
action d'un agent d'halogénation sur un composé cétone représenté par la formule (6) suivante : 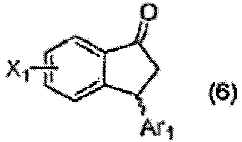 dans laquelle Ar₁ et X₁ représentent les mêmes groupes que ci-dessus, pour produire un composé représenté par la formule (7) suivante : 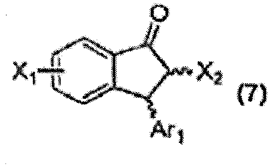 dans laquelle Ar₁ et X₁ représentent les mêmes groupes que ci-dessus ; et X₂ représente un atome d'halogène, et
réaction du composé (7) avec une base.

18. Méthode selon la revendication 17, dans laquelle l'agent d'halogénation est le brome.

19. Méthode selon l'une quelconque des revendications 17 ou 18, dans laquelle la base est d'une sorte ou plus choisie dans le groupe constitué de triéthylamine, diisopropyléthylamine, pyridine, carbonate de lithium, carbonate de sodium, carbonate de potassium, hydroxyde de lithium, hydroxyde de sodium, et hydroxyde de potassium.


### SEQUENCE LISTING

<110> Kaneka Corporation
<120> A Haloindenone and a Method for Producing Optically Active Indanone or Optically Active Indanol Using the Haloindenone
<150> JP2010-251095
   <151> 9 November 2011
<130> F11-066PCT
<160> 16
<170> PatentIn version 3.1
<210> 1
   <211> 30
   <212> DNA
   <213> Artificial
<220>
   <223> primer 1
<400> 1
   atcgagctct tatcagttct tgttccaacc 30
<210> 2
   <211> 35
   <212> DNA
   <213> Artificial
<220>
   <223> primer 2
<400> 2
   acgcgtcgac ttatcagttc ttgttccaac ctaaa 35
<210> 3
   <211> 40
   <212> DNA
   <213> Artificial
<220>
   <223> primer 3
<400> 3
   aatatataca tatgtcgttt atgaactttg aaccaaagcc 40
<210> 4
   <211> 44
   <212> DNA
   <213> Artificial
<220>
   <223> primer 4
<400> 4
   atatgagctc ttactatttc ttgtaaccct tggcaacagc ttcc 44
<210> 5
   <211> 39
   <212> DNA
   <213> Artificial
<220>
   <223> primer 5
<400> 5
   atatatacat atggccagaa aattatttac acctattac 39
<210> 6
   <211> 41
   <212> DNA
   <213> Artificial
<220>
   <223> primer 6
<400> 6
   atatgagctc ttattaccag cctctttcgt attgaacagg g 41
<210> 7
   <211> 45
   <212> DNA
   <213> Artificial
<220>
   <223> primer 7
<400> 7
   atagaattct aaggaggtta acaatgtcat ctgaaaaact gtatt 45
<210> 8
   <211> 40
   <212> DNA
   <213> Artificial
<220>
   <223> primer 8
<400> 8
   atatggtacc ttattacaac gtcgggtaat cggtatagcc 40
<210> 9
   <211> 40
   <212> DNA
   <213> Artificial
<220>
   <223> primer 9
<400> 9
   atatatacat atgtccgcac tgttcgaacc ctacaccctc 40
<210> 10
   <211> 45
   <212> DNA
   <213> Artificial
<220>
   <223> primer 10
<400> 10
   atatgagctc ttatcagcga taacgctcga gccagtgtgc ataag 45
<210> 11
   <211> 38
   <212> DNA
   <213> Artificial
<220>
   <223> primer 11
<400> 11
   atatatacat atgagcctgc tgctcgagcc ttacaccc 38
<210> 12
   <211> 43
   <212> DNA
   <213> Artificial
<220>
   <223> primer 12
<400> 12
   atatgagctc ttattaatcc cgcaagtccg actcatgtat cgg 43
<210> 13
   <211> 36
   <212> DNA
   <213> Artificial
<220>
   <223> primer 13
<400> 13
   atatatacat atgaagactg ctaaactgtt ctctcc 36
<210> 14
   <211> 40
   <212> DNA
   <213> Artificial
<220>
   <223> primer 14
<400> 14
   atatgagctc ttattacagc gttgggtaat cagtgtagcc 40
<210> 15
   <211> 44
   <212> DNA
   <213> Artificial
<220>
   <223> primer 15
<400> 15
   atagaattct aaggaggtta acaatgccta gcttgtttga tccc 44
<210> 16
   <211> 37
   <212> DNA
   <213> Artificial
<220>
   <223> primer 16
<400> 16
   atatggtacc ttatcaatcc ccaagcaaag gataatc 37